(19) Europäisches Patentamt
European Patent Office
Office européen des brevets

(11) Numéro de publication : **0 292 400 B1**

(12) # FASCICULE DE BREVET EUROPEEN

(45) Date de publication du fascicule du brevet : **02.01.92 Bulletin 92/01**

(21) Numéro de dépôt : **88401227.9**

(22) Date de dépôt : **20.05.88**

(51) Int. Cl.$^5$ : **C07D 498/10,** A61K 31/435, // (C07D498/10, 263:00, 221:00)

(54) **Dérivés du Spiro (4,5) décane, leur procédé de préparation et les compositons pharmaceutiques les renfermant.**

(30) Priorité : **22.05.87 FR 8707186**

(43) Date de publication de la demande : **23.11.88 Bulletin 88/47**

(45) Mention de la délivrance du brevet : **02.01.92 Bulletin 92/01**

(84) Etats contractants désignés : **AT BE CH DE ES FR GB GR IT LI LU NL SE**

(56) Documents cités : **FR-A- 2 117 894**

(73) Titulaire : **ADIR ET COMPAGNIE
1 rue Carle Hébert
F-92415 Courbevoie Cédex (FR)**

(72) Inventeur : **Regnier, Gilbert
27 avenue du Plessis
F-92290 Chatenay Malabry (FR)**
Inventeur : **Guillonneau, Claude
21 rue des Bergers
F-92140 Clamart (FR)**
Inventeur : **Duhault, Jacques
14 bis rue Paul Demange
F-78290 Croissy S/Seine (FR)**
Inventeur : **Lonchampt, Michel
80 rue H. Crette Clos de Ricbourg Chevilly Larue
F-94150 Rungis (FR)**

(74) Mandataire : **Reverbori, Marcelle ADIR ET COMPAGNIE
1, rue Carle Hébert
F-92415 Courbevoie Cédex (FR)**

EP 0 292 400 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet les nouveaux dérivés du spiro (4,5) décane, leur procédé de préparation et les compositions pharmaceutiques les renfermant.

Elle concerne particulièrement les dérivés du spiro (4,5) décane de formule générale I :

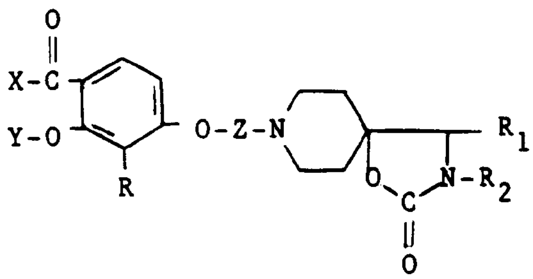

(I)

dans laquelle :

X représente un radical méthyle ; et

Y représente :

un atome d'hydrogène, ou un radical de formule :

R'-CO—

dans laquelle R' représente :

un radical alkyle ou alkoxy contenant chacun de 1 à 4 atomes de carbone, ou un radical (méthyl ou éthyl)-oxycarbonyle

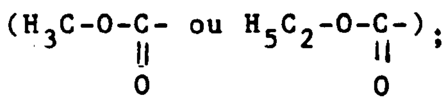

ou X et Y représentent ensemble une chaîne éthénylène de formule

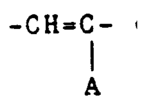

ou éthylène de formule :

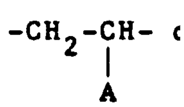

dans lesquelles A représente un atome d'hydrogène, un radical méthyle ou un groupe carboxy, méthoxycarbonyle ou éthoxycarbonyle ;

R représente un radical alkyle contenant de 1 à 7 atomes de carbone en chaîne droite ou ramifiée, comportant éventuellement une double liaison ;

Z est une chaîne polyméthylénique $(CH_2)_n$, n étant un nombre entier de 2 à 7, éventuellement ramifiée ou porteuse d'un radical hydroxy ;

$R_1$ représente un atome d'hydrogène ou un radical méthyle ; et

$R_2$ représente :

un atome d'hydrogène, un radical acyle de formule R'-CO- dans laquelle R' a la signification précédemment définie, ou un radical benzyloxycarbonyle.

L'état antérieur de la technique dans ce domaine est illustré notamment par le brevet FR n° 1.441.575 et le BSM correspondant n° 4463M qui ont respectivement pour objet les dérivés du spiro (4,5) décane de formule

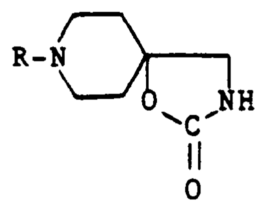

dans laquelle R représente, entre autres, les radicaux phénéthyle et phénoxyéthyle, et leur utilisation comme médicaments à propriétés analgésiques, anti-inflammatoires et broncholytiques.

Les dérivés de formule générale I se différencient de ceux de l'Art antérieur, non seulement par leur structure chimique mais aussi par leur comportement pharmacologique, comme le prouve l'étude pharmacologique ci-après exemplifiée.

La présente invention a également pour objet le procédé de préparation des dérivés de formule générale I, basé essentiellement sur la réaction :

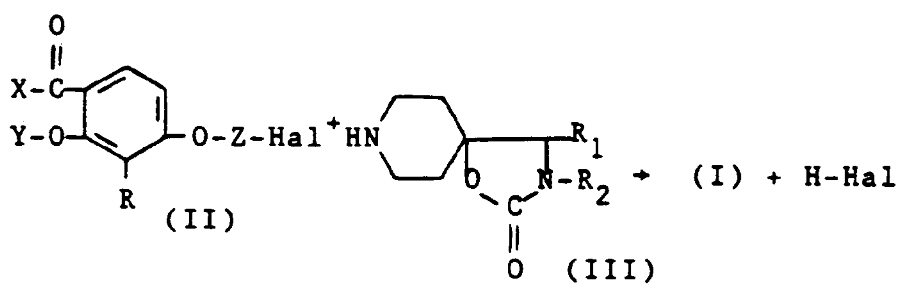

(Hal étant un atome d'halogène, préférentiellement un atome de brome). D'une manière générale, la condensation des dérivés II et III est toujours effectuée à une température comprise entre 60 et 100°C, en opérant dans un solvant adéquat tel que l'acétonitrile ou une cétone aliphatique contenant 3 ou 4 atomes de carbone, en présence d'un accepteur de l'hydracide formé au cours de la réaction. Comme accepteur on peut employer préférentiellement un excès du dérivé III ou, à défaut, une base tertiaire telle que la triéthylamine, ou un carbonate alcalin comme $K_2CO_3$ ou $Na_2CO_3$.

Ce procédé de base s'accompagne d'un certain nombre de variantes selon les produits à préparer et plus précisément selon les significations prises par X, Y et $R_2$. Toutes ces variantes sont incluses dans la présente invention.

C'est ainsi que :

1. Dans le cas où X représente un radical méthyle :

1.1. Pour obtenir les dérivés de formule I dans laquelle Y et $R_2$ représentent simultanément un atome d'hydrogène, on effectue la condensation du dérivé de formule générale II a :

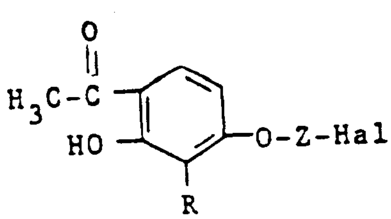

(IIa)

dans laquelle R, Z et Hal ont les significations énoncées précédemment,

avec un dérivé de formule générale IIIa

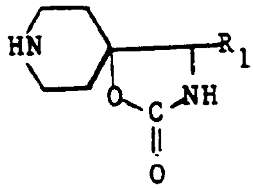

(IIIa)

dans laquelle $R_1$ a la signification précédemment définie, qui conduit aux dérivés de formule générale I a

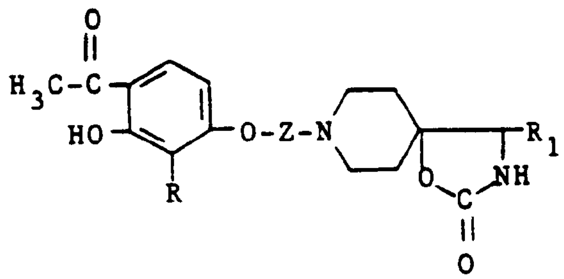

(Ia)

dans laquelle R, Z et $R_1$ ont les significations précédemment définies.

1.2. Pour obtenir les formes acylées des dérivés I alternativement ou simultanément sur les sites Y et $R_2$, on fait réagir un dérivé de formule générale Ia avec une forme réactive de l'agent acylant tel que par exemple un chlorure d'acide R'COCl ou un anhydride $(R'COO)_2O$, (R' ayant dans ces formules la signification précédemment définie), en tenant compte du fait que l'acylation se fait d'abord sur le site $R_2$ et ensuite sur le site Y. Ainsi, en faisant réagir un dérivé de formule générale Ia avec la quantité stoechiométrique d'agent acylant de formule R'COCl ou $(R'COO)_2O$ on obtient la forme diacylée de formule générale Ib :

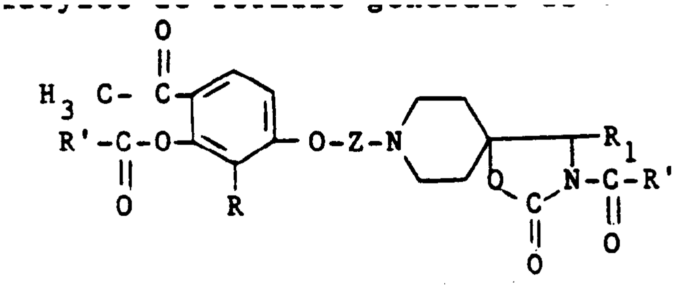

(Ib)

Une telle acylation s'effectue de manière particulièrement avantageuse, en opérant en présence d'une base tertiaire telle par exemple la diméthylamino-4 pyridine, dans un solvant halogéné tel que par exemple le chlorure de méthylène, à température ambiante.

En faisant réagir un dérivé de formule générale Ia avec la moitié de la quantité stoechiométrique de l'agent acylant de formule R'COCl ou $(R'COO)_2O$ on obtient la forme monoacylée sur le site $R_2$, de formule générale Ic :

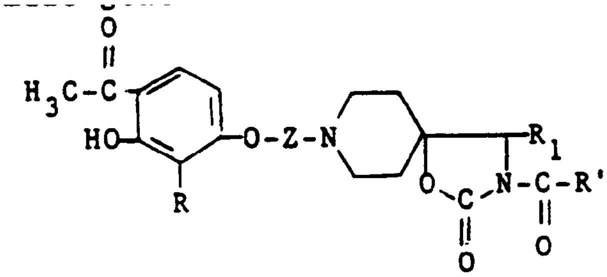

(Ic)

Enfin pour obtenir la forme monoacylée des dérivés I sur le site Y, exclusivement, on protègera le site $R_2$ avant

d'effectuer l'acylation pour ensuite supprimer la protection du site $R_2$ selon les méthodes classiques, c'est-à-dire que : l'on fait réagir sur le dérivé (Ia) le dérivé de formule

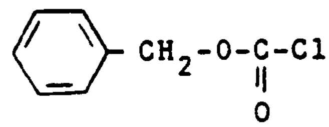

pour obtenir le dérivé de formule générale Id :

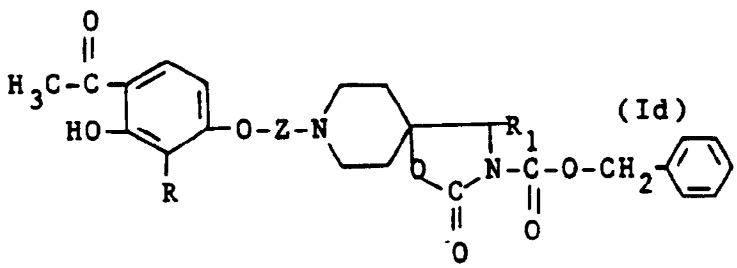

(Id)

dans laquelle R, Z et $R_1$ ont les significations précédemment définies, lequel dérivé (Id) est acylé selon la méthode précédemment décrite pour la diacylation au moyen de R'COCl ou $(R'COO)_2O$ pour obtenir le dérivé de formule générale Ie :

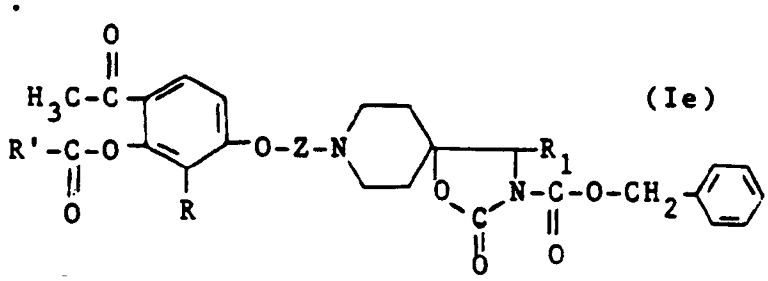

(Ie)

dans laquelle R, R' Z et $R_1$ ont les significations précédemment définies, sur lequel on effectue la déprotection du site $R_2$ par hydrogénolyse du groupement benzyloxycarbonyle, pour obtenir un dérivé de formule générale If :

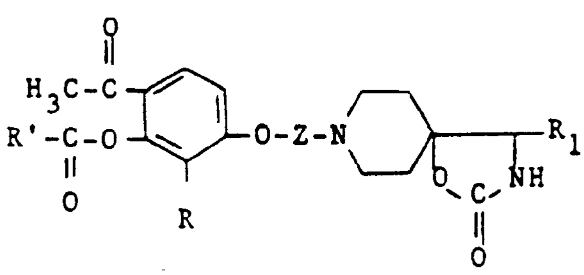

(If)

L'hydrogénolyse du groupe benzyloxycarbonyle du dérivé (Ie) s'effectue avantageusement au moyen d'hydrogène à pression ordinaire en présence de charbon palladié comme catalyseur dans un solvant polaire tel que par exemple l'éthanol ou le méthanol.

2. Dans le cas où X et Y représentent ensemble une chaîne éthyényle ou éthylène substituée par un groupe carboxy il convient de condenser le dérivé (III), sur une forme estérifiée, par un reste méthyle ou éthyle, du dérivé (II) correspondant, puis de saponifier le dérivé obtenu au moyen de $NaHCO_3$ pour libérer la fonction carboxy.

Tous les produits de formule générale I sont des bases faibles qui peuvent à ce titre former des sels avec

des acides minéraux ou organiques biologiquement compatibles. Ces sels sont également inclus dans la présente invention.

Les produits, de formule générale I peuvent être purifiés par chromatographie flash sur colonne de silice (35-70 μ) avec des systèmes $CH_2Cl_2/CH_3OH$, ou par cristallisation dans des solvants appropriés.

Les matières premières utilisées pour préparer les dérivés de formule générale I sont soit des produits connus soit des produits préparés selon un procédé connu pour la préparation de composés analogues, comme mentionné dans le tableau ci-après :

| matières premières | Référence de préparation |
|---|---|
| 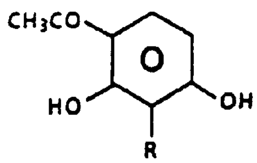 | (R=allyl, n-propyl)<br>W. BAKER et O.M. LOTHIAN<br>J. Chem. Soc. (1935)628-633 |
| 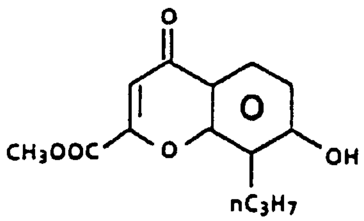 | R.A. APPLETON et coll.<br>J.Med. chem. (1977)20,371-378 |
| 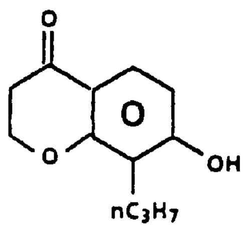 | Analogie avec D. HUCKLE et coll.<br>J.Med. Chem.(1969) 12,277 |
| 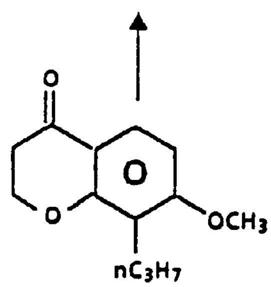 | Analogie avec B.GRAFFE et coll.<br>J.Hetero Chem.(1975) 12,247-251 |
| 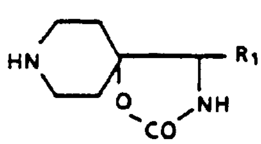 | $R_1$=H Science Union et Cie<br>Brevet français 1 441 575<br>$R_1$=$CH_3$ J. MAILLARD et coll.<br>Ch.therap.(1973) 393-397 |
| 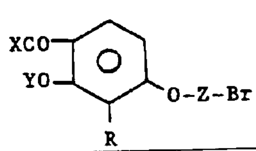 | Analogie avec ELLI LILLY<br>EP 0108592 A1 du 16 mai 1984 |

Pour les matières premières de formule générale II dans laquelle Hal représente un atome de brome, ont été préparés les produits suivants :

| 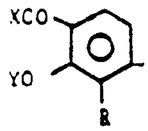 | Z | PF* (kofler) |
|---|---|---|
| 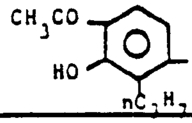 | $-(CH_2)_2-$ | huile |
| 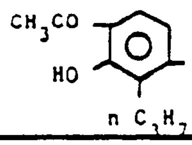 | $-(CH_2)_3-$ | huile |
| 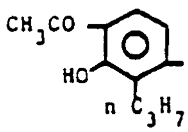 | $-(CH_2)_4-$ | huile |
| 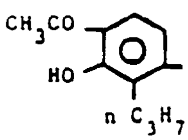 | $-(CH_2)_5-$ | huile |
| 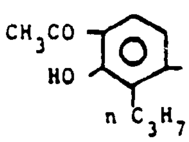 | $-(CH_2)_6-$ | huile |
| 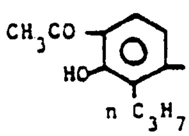 | $CH_2-CHOH-CH_2$ | huile |
| 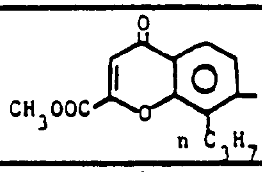 | $-(CH_2)_3-$ | 110°C |
| 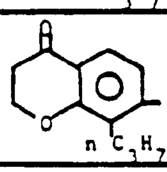 | $-(CH_2)_3-$ | 64°C |
| 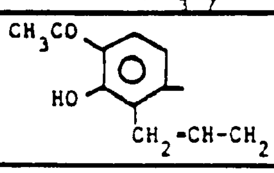 | $-(CH_2)_3-$ | huile |

* tous ces composés ont été purifiés par chromatographie flash sur colonne $SiO_2$(35-70 µ) avec le système $CH_2Cl_2$-cyclohexane (50-50). Les spectres RMN sont conformes avec les structures proposées.

Les dérivés de formule générale I et leurs sels physiologiquement tolérables possèdent des propriétés pharmacologiques et thérapeutiques intéressantes notamment des propriétés anti-bronchoconstricteur, antiasthmatique, anti-allergique et anti-inflammatoire bronchique ; qui, de ce fait permettent leur utilisation comme médicament notamment dans le traitement de l'asthme et de ses complications à long terme, la prévention et le traitement des phénomènes allergiques, en particulier les bronchoconstrictions précoces et tardives, le traitement des bronchopathies chroniques obstructives, de l'hypertension artérielle pulmonaire, des manifestations respiratoires de diverses maladies entraînant inflammation et oedème de l'arbre tracheobronchique et de l'interstitium pulmonaire, et des affections inflammatoires des voies respiratoires hautes.

La présente invention a pour objet les compositions pharmaceutiques contenant comme principe actif un composé de formule générale I ou un de ses sels physiologiquement tolérable mélangé ou associé à un excipient pharmaceutique approprié comme par exemple, l'eau distillée, le glucose, le lactose, l'amidon, le talc, l'éthyl cellulose, le stéarate de magnésium ou le beurre de cacao.

Les compositions pharmaceutiques ainsi obtenues se présentent généralement sous forme dosée et peuvent contenir de 20 à 80 mg de principe actif. Elles peuvent revêtir, par exemple, la forme de comprimés, dragées, gélules, suppositoires, solutions injectables ou buvables, et être selon les cas administrées par voie orale, rectale ou parentérale à la dose de 20 à 80 mg 1 à 3 fois par jour.

Les exemples suivantes illustrent l'invention, les points de fusion étant, sauf mention contraire, déterminés au tube capillaire.

## EXEMPLE 1 :

[(Propyl-2 hydroxy-3 acétyl-4 phénoxy)-3 propyl]-8 oxa-1 oxo-2 diaza-3,8 spiro (4,5) décane.

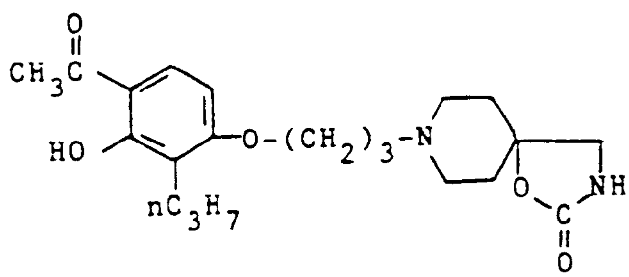

On chauffe pendant 15 h. à reflux une solution contenant 8 g (0,025 mol) de (bromo-3 propoxy)-4 n.propyl-3 hydroxy-2 acétophénone (huile), 7,9 g (0,05 mol) d'oxa-1 oxo-2 [8H] diaza-3,8 spiro (4,5) décane avec 0,2 g d'iodure de sodium dans 160 ml d'acétonitrile. Ensuite, on évapore le solvant sous pression réduite et traite le résidu avec 80 ml d'eau et 50 ml de $CH_2Cl_2$ ; on décante la couche aqueuse, lave à l'eau la couche organique et l'évapore sous pression réduite. Le résidu semi-cristallin est purifié par chromatographie flash sur une colonne contenant 450 g de $SiO_2$ en éluant avec le système $CH_2Cl_2$-$CH_3OH$ (95-5) sous une pression d'azote de 0,5 atmosphère. Après évaporation des éluants, on obtient 8,8 g de cristaux blancs fondants à 112-114°C de [(propyl-2 hydroxy-3 acétyl-4 phénoxy-3 propyl]-8 oxa-1 oxo-2 diaza-3,8 spiro (4,5) décane.

De la même façon ont été préparés les produits suivants :

a) (Propyl-2 hydroxy-3 acétyl-4 phénoxy)-2 éthyl-8 oxa-1 oxo-2 diaza-3,8 spiro (4,5) décane, P.F. 128-130°C.

b) [(Propyl-2 hydroxy-3 acétyl-4 phénoxy)-4 butyl]-8 oxa-1 oxo-2 diaza-3,8 spiro (4,5) décane, P.F. 112-114°C.

c) [(Propyl-2 hydroxy-3 acétyl-4 phénoxy)-3 hydroxy-2 propyl]-8 oxa-1 oxo-2 diaza-3,8 spiro (4,5) décane, P. F. 145-147°C.

d) [(Propyl-2 hydroxy-3 acétyl-4 phénoxy)-5 pentyl]-8 oxa-1 oxo-2 diaza-3,8 spiro (4,5) décane, P.F. 118-120°C.

e) [(Propyl-2 hydroxy-3 acétyl-4 phénoxy)-6 hexyl]-8 oxa-1 oxo-2 diaza-3,8 spiro (4,5) décane, P.F. 136-138°C.

f) [(Propyl-2 hydroxy-3 acétyl-4 phénoxy)-2 éthyl]-8 oxa-1 oxo-2 diaza-3,8 méthyl-4 spiro (4,5) décane, P.F. 114-116°C.

g) [(Propyl-2 hydroxy-3 acétyl-4 phénoxy)-3 propyl]-8 oxa-1 oxo-2 diaza-3,8 méthyl-4 spiro (4,5) décane P.F. 83-85°C.

h) [(Allyl-2 hydroxy-3 acétyl-4 phénoxy)-3 propyl]-8 oxa-1 oxo-2 diaza-3,8 spiro (4,5) décane, P.F. 134-136°C (isopropanol)

i) [(Ethoxycarbonyl-2 propyl-8 chormone-4-yl-7 oxy)-3 propyl]-8 oxa-1 oxo-2 diaza-3,8 spiro (4,5) décane, P.F. 152-154°C.

j) [(Oxo-4 propyl-7 chromanyl-7 oxy)-3 propyl]-8 oxa-1 oxo-2 diaza-3,8 spiro (4,5) décane, P.F. 124-126°C.

k) [(Ethyl-2 hydroxy-3 acétytl-4 phénoxy)-3 propyl]-8 oxa-1 oxo-2 diaza-3,8 spiro (4,5) décane, P.F. 150-152°C (isopropanol)

l) [(Isobutyl-2 hydroxy-3 acétyl-4 phénoxy)-3 propyl]-8 oxa-1 oxo-2 diaza-3,8 spiro (4,5) décane P.F. 130-132°C. (isopropanol)

**EXEMPLE 2 :**

[(Propyl-2 hydroxy-3 acétyl-4 phénoxy)-3 propyl]-8 oxa-1 oxo-2 diaza-3,8 méthyl-4 spiro (4,5) décane.

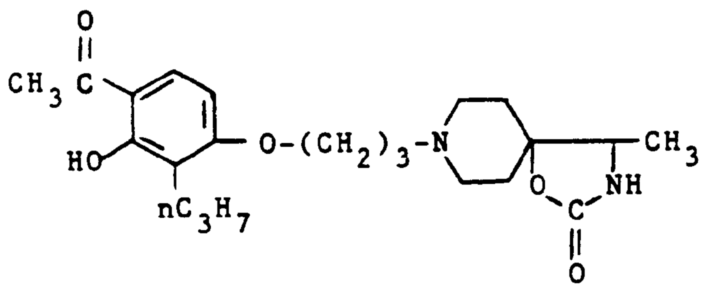

On chauffe pendant 15 h à reflux une solution de 8 g (0,025 mol) de (bromo-3 propoxy)-4 n.propyl-3 hydroxy-2 acétophénone avec 4,25 g (0,025 mol) d'oxa-1 oxo-2 méthyl-4 [8H] diaza-3,8 spiro (4,5) décane, en présence de 3,45 g (0,025 mol) de $K_2CO_3$ et 0,2 g d'iodure de sodium. Ensuite, on traite comme dans l'exemple 1 et obtient finalement 80 g de [(propyl-2 hydroxy-3 acétyl-4 phénoxy)-3 propyl]-8 oxa-1 oxo-2 diaza-3,8 méthyl-4 spiro (4,5) décane, sous forme de cristaux blancs, fondant à 83-85°C. De la même façon, ont été préparés tous les produits objet des exemples 1 et 1a à 11.

**EXEMPLE 3 :**

[(Carboxy-2 propyl-8 chromone-4 yl-7) oxy-3 propyl]-8 oxa-1 oxo-2 diaza-3,8 spiro (4,5) décane.

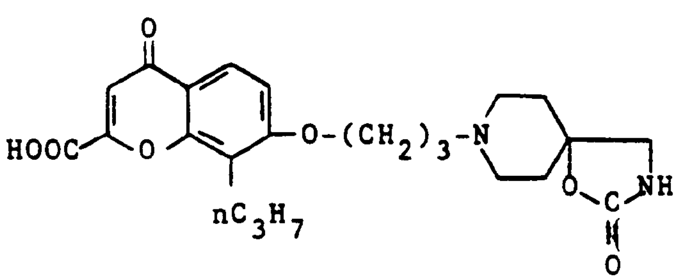

On chauffe pendant 1 h 30 à reflux une solution de 7,1 g (0,015 mol) de [(carbéthoxy-2 propyl-8 chromone-4yl-7) oxy-3 propyl]-8 oxa-1 oxo-2 diaza-3,8 spiro (4,5) décane fondant à 152-154°C préparé selon l'exemple 1, dans 3500 ml d'éthanol contenant 6,3 g de $NaHCO_3$ dissout dans 35 ml d'eau. Ensuite, on évapore le solvant sous pression réduite et neutralise exactement par addition de 75 ml de HCl,N. Il y a solubilisation totale suivie d'une précipitation. On filtre le produit, lave à l'eau et a l'éther. Après séchage, on obtient 6,5 g de produit blanc. On le reprend par 15 ml d'eau à reflux et ajoute 30 ml d'HCl,N : il y a dissolution totale puis recristallisation. On refroidit, essore et sèche sous vide. On obtient finalement 6 g de cristaux de chlorhydrate de [(carboxy-2 propyl-8 chromone-4 yl-7) oxy-3 propyl]-8 oxa-1 oxo-2 diaza-3,8 spiro (4,5) decane, fondant vers 180-200°C en se décomposant.

**EXEMPLE 4 :**

[(Propyl-2 acétoxy-3 acétyl-4 phénoxy)-3 propyl]-8 oxa-1 oxo-2 diaza-3,8 acétyl-3 spiro (4,5) décane.

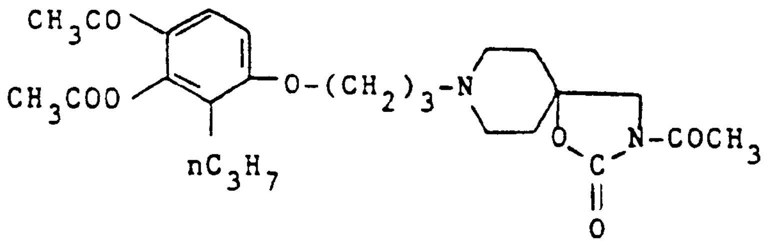

A une solution de 7,78 g (0,02 mol) de [(propyl-2 hydroxy-3 acétyl-4 phénoxy)-3 propyl]-8 oxa-1 oxo-2 diaza-3,8 spiro (4,5) décane, préparé selon l'exemple 1, dans 100 ml de $CH_2Cl_2$ contenant 5,4 g (0,044 mol) de diméthylamino-4-pyridine, on ajoute 40 ml d'anhydride acétique en solution dans 100 ml de $CH_2Cl_2$ puis agite la solution pendant 4 h à température ordinaire. Ensuite, on évapore à sec et reprend le résidu par 50 ml de $CH_2Cl_2$ et 50 ml de $NaHCO_3$ à 10%. Après décantation, on sèche le $CH_2Cl_2$ sur $Na_2SO_4$, évapore la solution et purifie le résidu par chromato flash sur colonne contenant 450 g de $SiO_2$ sous une pression d'azote de 0,5 atmosphère en éluant avec le système $CH_2$-$CH_3OH$ (97-3). Après évaporation des éluats, on obtient finalement 6,1 g de base amorphe qu'on transforme en fumarate au sein de l'éthanol. On isole 6,8 g de cristaux de fumarate acide de [(propyl-2 acétoxy-3 acétyl-4 phénoxy)-3 propyl]-8 oxa-1 oxo-2 diaza-3,8 acétyl-3 spiro (4,5) décane, fondant à 144-146°C.

De la même façon ont été préparés les produits suivants :

a) [(Allyl-2 acétoxy-3 acétyl-4 phénoxy)-3 propyl]-8 oxa-1 oxo-2 diaza-3,8 acétyl-3 spiro (4,5) décane, P.F. 130-132°C.

b) [(Propyl-2 acétoxy-3 acétyl-4 phénoxy)-5 pentyl]-8 oxa-1 oxo-2 diaza-3,8 acétyl-3 spiro (4,5) décane, P.F. de fumarate acide correspondant : 158-160°C (isopropanol)

c) [(Ethyl-2 acétoxy-3 acétyl-4 phénoxy)-3 propyl]-8 oxa-1 oxo-2 diaza-3,8 acétyl-3 spiro (4,5) décane, P.F. 135-136°C. (éthanol)

d) [(Isobutyl-2 acétoxy-3 acétyl-4 phénoxy)-3 propyl]-8 oxa-1 oxo-2 diaza-3,8 acétyl-3 spiro (4,5) décane, P.F. de fumarate acide correspondant : 162-164°C (isopropanol)

**EXEMPLE 5 :**

[(Allyl-2 hydroxy-3 acétyl-4 phénoxy)-3 propyl]-8 oxa-1 oxo-2 diaza-3,8 acétyl-3 spiro (4,5) décane.

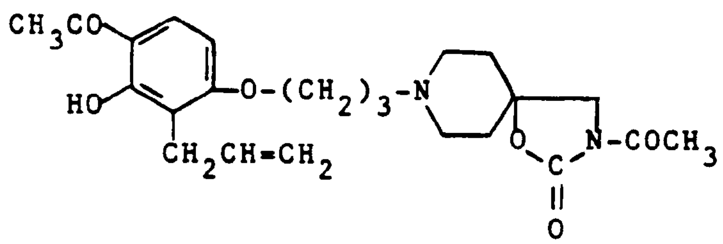

En opérant comme dans l'exemple 4, à partir de 3,9 g (0,01 mol) d' [(allyl-2 hydroxy-3 acétyl-4 phénoxy)-3 propyl]-8 oxa-1 oxo-2 diaza-3,8 spiro (4,5) décane, fondant à 134-136°C et préparé selon l'exemple 1, et de 0,11 mol d'anhydride acétique, on obtient finalement 2,1 g d' [(allyl-2 hydroxy-3 acétyl-4 phénoxy)-3 propyl]-8 oxa-1 oxo-2 diaza-3,8 acétyl-3 spiro (4,5) décane, sous forme de cristaux blancs fondants à 100-102°C.

**EXEMPLE 6 :**

[(Propyl-2 acétoxy-3 acétyl-4 phénoxy)-3 propyl]-8 oxa-1 oxo-2 diaza-3,8 spiro (4,5) décane.

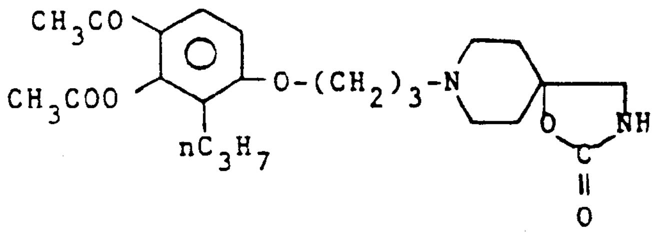

On prépare d'abord, selon l'exemple 5, le [(propyl-2 hydroxy-3 acétyl-4 phénoxy)-3 propyl]-8 oxa-1 oxo-2 diaza-3,8 carbobenzoxy-3 spiro (4,5) décane, fondant à 112°C, avec un rendement de 50% à partir du composé décrit dans l'exemple 1 et du chloroformiate de benzyle.

Le composé obtenu est ensuite transformé avec un rendement de 87% selon l'exemple 4, en [(propyl-2 acétoxy-3 acétyl-4 phénoxy)-3 propyl]-8 oxa-1 oxo-2 diaza-3,8 benzyloxycarbonyl-3 spiro (4,5) décane, fondant à 160-162°C, en utilisant un excès d'anhydride acétique en présence de diméthylamino-4 pyridine. Une solution de 9,2 g (0,016 mol) du composé précédent en solution dans 800 ml d'éthanol à 90% est hydrogénée sous 0,3 atmosphère d'hydrogène en présence de 0,8 g de Pd/C à 5% et de 16,3 ml HCl, N. Lorsque la quantité d'hydrogène a été absorbée, on filtre le catalyseur et évapore à sec. Le résidu cristallin est recristallisé dans 300 ml d'éthanol en présence de 2 ml d'éther, HCl(pH : 2). On recueille 6,7 g de chlorhydrate de [(propyl-2 acétoxy-3 acétyl-4 phénoxy)-3 propyl]-8 oxa-1 oxo-2 diaza-3,8 spiro (4,5) décane, fondant à 233-235°C.

De la même façon a été préparé le dérivé suivant :

a)[(Propyl-2 acétoxy-3 acétyl-4 phénoxy)-5 pentyl]-8 oxa-1 oxo-2 diaza-3,8 spiro (4,5) décane, P.F. du fumarate acide correspondant 120-122°C. (isopropanol)

**EXEMPLE 7 :**

Etude pharmacologique des dérivés de formule générale I. Les dérivés de l'invention et le fenspiride, produit du Prior Art pris comme substance de référence, ont été soumis au test de dégranulation des mastocytes:

Des mastocytes péritonéaux de rats sont mis à incuber in vitro en présence du composé 48/80 qui provoque la libération de granules d'histamine. L'effet protecteur des composés testés est exprimé en $IC_{50}$ c'est-à-dire la dose (molaire) qui inhibe de 50% cette libération.

Les résultats obtenus sont répertoriés dans le tableau suivant :

| Composés des Exemples | Dégranulation des mastocytes in vitro - $IC_{50}$ (mole). |
|---|---|
| 1 | $2,4.10^{-4}$ |
| 1a | $2.10^{-4}$ |
| 1b | $2.10^{-4}$ |
| 1c | $10^{-3}$ |
| 1d | $1,1.10^{-4}$ |
| 1e | $9,2.10^{-5}$ |
| 1f | $10^{-4}$ |
| 1g | $2.10^{-4}$ |
| 1h | $2,5.10^{-4}$ |
| 1i | $10^{-4}$ |
| 1j | $10^{-4}$ |
| 1k | $8,3.10^{-4}$ |
| 1l | $1,16.10^{-4}$ |
| 2 | $2.10^{-4}$ |
| 3 | $10^{-3}$ |
| 4 | $7,2.10^{-4}$ |
| 4a | $2,5.10^{-3}$ |
| 4b | $10^{-3}$ |
| 4c | $> 10^{-3}$ |
| 4d | $7,3.10^{-4}$ |
| 5 | $7,5.10^{-4}$ |
| 6 | $1,2.10^{-3}$ |
| 6a | $2.10^{-3}$ |
| fenspiride | inactif |

L'examen de ce tableau montre la supériorité des dérivés de la présente invention sur le produit le plus proche du Prior Art qui lui est inactif sur le test de dégranulation des mastocytes.

Par ailleurs, la toxicité des dérivés de la formule générale I est faible. La dose toxique moyenne déterminée chez la souris par voie intrapéritonéale est pour ces dérivés du même ordre de grandeur que celle du fenspiride.

La faible toxicité et l'activité importante des présents dérivés permettent donc leur utilisation en thérapeutique notamment dans le domaine respiratoire.

## Revendications

### Revendications pour les Etats contractants suivants : AT, BE, CH, DE, FR, GB, IT, LI, LU, NL, SE

1. Les dérivés du spiro (4,5) décane de formule générale I :

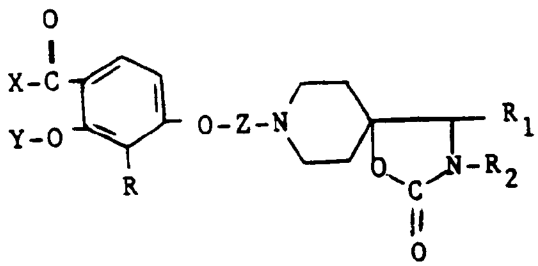

(I)

dans laquelle :

X représente un radical méthyle ; et

Y représente :

un atome d'hydrogène, ou un radical de formule :

R'-CO

dans laquelle R' représente :

un radical alkyle ou alkoxy contenant chacun de 1 à 4 atomes de carbone, ou un radical (méthyl ou éthyl)-oxycarbonyle

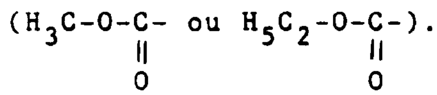

ou X et Y représentent ensemble une chaîne éthénylène de formule

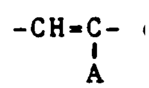

ou éthylène de formule :

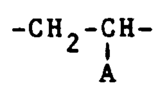

dans lesquelles A représente un atome d'hydrogène, un radical méthyle ou un groupe carboxy, méthoxycarbonyle ou éthoxycarbonyle.

R représente un radical alkyle contenant de 1 à 7 atomes de carbone en chaîne droite ou ramifiée, comportant éventuellement une double liaison ;

Z est une chaîne polyméthylénique $(CH_2)_n$, n étant un nombre entier de 2 à 7, éventuellement ramifiée ou porteuse d'un radical hydroxy ;

$R_1$ représente un atome d'hydrogène ou un radical méthyle ; et

$R_2$ représente :

un atome d'hydrogène, un radical acyle de formule R'-CO- dans laquelle R' a la signification précédemment

définie, ou un radical benzyloxycarbonyle.

2. Les sels des dérivés de la revendication 1 avec des acides biologiquement compatibles.

3. Le [(propyl-2 hydroxy-3 acétyl-4 phénoxy)-3 propyl]-8 oxa-1 oxo-2 diaza-3,8 spiro (4,5) décane.

4. Le [(propyl-2 hydroxy-3 acétyl-4 phénoxy)-5 pentyl]-8 oxa-1 oxo-2 diaza-3,8 spiro (4,5) décane.

5. Le [(propyl-2 hydroxy-1 acétyl-4 phénoxy)-6 hexyl]-8 oxa-1 oxo-2 diaza-3,8 spiro (4,5) décane.

6. Le [(propyl-2 acétoxy-3 acétyl-4 phénoxy)-3 propyl]-8 oxa-1 oxo-2 diaza-3,8 acétyl-3 spiro (4,5) décane.

7. L' [(allyl-2 acétoxy-3 acétyl-4 phénoxy)-3 propyl]-8 oxa-1 oxo-2 diaza-3,8 acétyl-3 spiro (4,5) décane.

8. Le [(propyl-2 acétoxy-3 acétyl-4 phénoxy)-3 propyl]-8 oxa-1 oxo-2 diaza-3,8 spiro (4,5) décane.

9. Le procédé de préparation des dérivés de la revendication 1 caractérisé en ce que l'on fait réagir un dérivé halogéné de formule générale II :

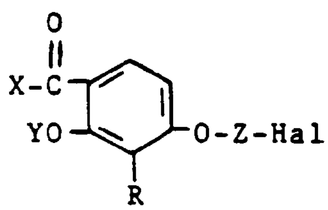

(II)

dans laquelle X, Y, R et Z ont les significations définies dans la revendication 1 et Hal représente un atome d'halogène, avec un dérivé de formule générale III :

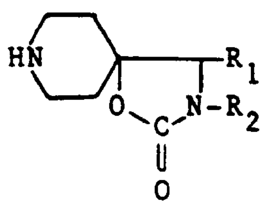

(III)

dans laquelle $R_1$ et $R_2$ sont tels que définis dans la revendication 1.

10. Le procédé de préparation des dérivés de formule générale Ia :

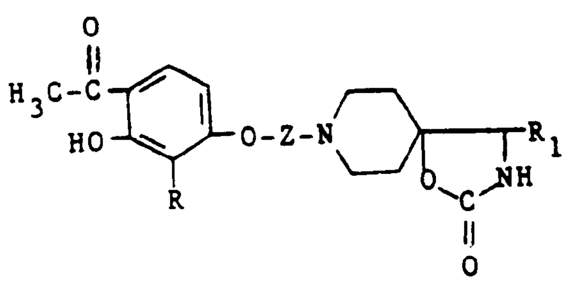

(Ia)

dans laquelle R, Z, et $R_1$ ont les significations définies dans la revendication 1, caractérisé en ce que : l'on effectue la condensation d'un dérivé de formule générale IIa :

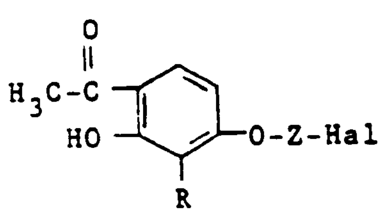

(IIa)

dans laquelle R, Z et Hal sont tels que définis dans la revendication 9, avec un dérivé de formule générale IIIa:

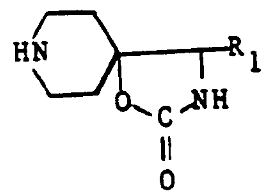

(IIIa)

dans laquelle $R_1$ a la signification énoncée dans la revendication 9.

11. Le procédé de préparation des dérivés de formule générale Ib :

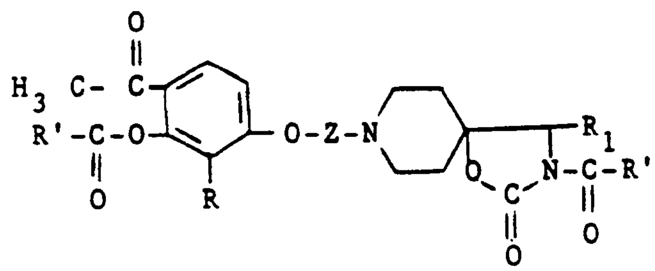

(Ib)

dans laquelle R, R′, Z et $R_1$ ont les significations définies dans la revendication 1, caractérisé en ce que l'on fait réagir un dérivé de formule générale Ia selon la revendication 10, avec la quantité stoechiométrique d'agent acylant de formule :

R′COCl ou $(R'COO)_2O$,

dans lesquelles R′ a la signification définie dans la revendication 1.

12. Le procédé de préparation des dérivés de formule générale Ic :

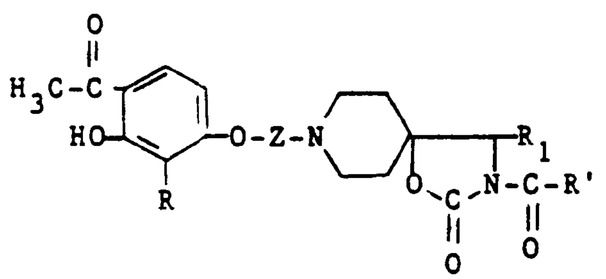

(Ic)

dans lesquelle R, Z, R′ et $R_1$ ont les significations définies dans la revendication 1, caractérisé en ce que l'on fait réagir un dérivé de formule générale Ia selon la revendication 10, avec la moitié de la quantité stoechiométrique d'agent acylant R′COCl ou $(R'COO)_2O$ tel que défini dans le revendication 11.

13. Le procédé de préparation des dérivés de formule générale Id, Ie et If ci-après décrites, caractérisé en ce que :

a) l'on fait réagir sur le dérivé Ia défini dans la revendication 10, un dérivé de formule :

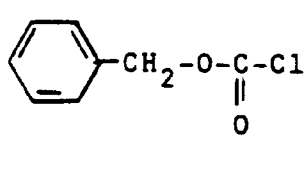

pour obtenir le dérivé de formule générale Id :

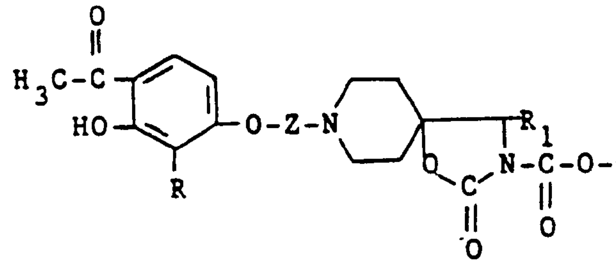

(Id)

dans laquelle R, Z et $R_1$ ont les significations définies dans la revendication 1 ;

b) l'on acyle le dérivé (Id) ainsi obtenu avec un agent acylant R'COCl ou $(R'COO)_2O$ selon la revendication 11, pour obtenir le dérivé de formule générale Ie :

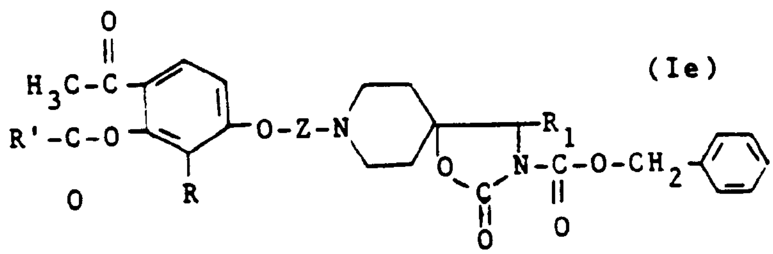

(Ie)

dans laquelle R, R', Z et $R_1$ ont les significations définies dans la revendication 1 ; et

c) l'on hydrogénolyse le dérivé Ie ainsi obtenu au moyen d'hydrogène, en présence de charbon palladié comme catalyseur, pour obtenir le dérivé de formule générale If :

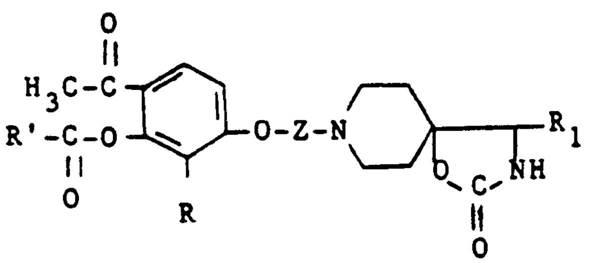

(If)

dans laquelle R, R', Z et $R_1$ ont les significations définies dans la revendication 1.

14. Le procédé de préparation des dérivés de la revendication 1 répondant à la formule générale I dans laquelle X et Y représentent ensemble une chaîne éthényle ou éthylène substituée par un groupe carboxy, caractérisé en ce que l'on condense le dérivé III selon la revendication 9, sur une forme estérifiée, par un reste méthyle ou éthyle, du dérivé II correspondant selon la revendication 9, puis l'on saponifie le dérivé obtenu au moyen de $NaHCO_3$ pour libérer la fonction carboxy.

15. Les compositions pharmaceutiques contenant comme principe actif un dérivé selon les revendications 1 à 8, avec des excipients pharmaceutiques appropriés.

16. Les compositions pharmaceutiques selon la revendication 15 présentées sous une forme convenant notamment pour le traitement de l'asthme, des phénomènes allergiques, des bronchopathies chroniques obstructives, de l'hypertension artérielle pulmonaire et des affections inflammatoires des voies respiratoires hautes.

**Revendications pour l'Etat contractant suivant : ES**

1. Le procédé de préparation des dérivés du spiro (4,5) décane de formule générale I :

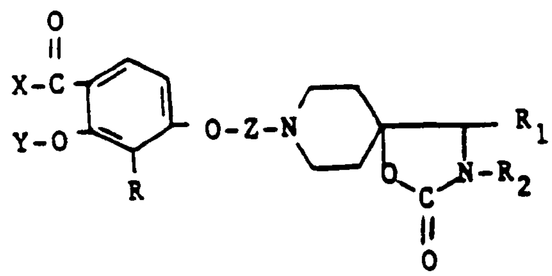

(I)

dans laquelle :

X représente un radical méthyle ; et

Y représente :

un atome d'hydrogène, ou un radical de formule :

R'-CO—

dans laquelle R' représente :

un radical alkyle ou alkoxy contenant chacun de 1 à 4 atomes de carbone, ou un radical (méthyl ou éthyl)-oxycarbonyle

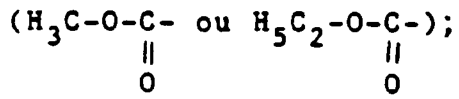

ou X et Y représentent ensemble une chaîne éthénylène de formule

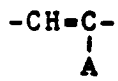

ou éthylène de formule :

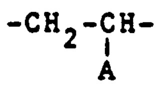

dans lesquelles A représente un atome d'hydrogène, un radical méthyle ou un groupe carboxy, méthoxy-carbonyle ou éthoxycarbonyle.

R représente un radical alkyle contenant de 1 à 7 atomes de carbone de chaîne droite ou ramifiée, comportant éventuellement une double liaison ;

Z est une chaîne polyméthylénique $(CH_2)_n$, n étant un nombre entier de 2 à 7, éventuellement ramifiée ou porteuse d'un radical hydroxy ;

$R_1$ représente un atome d'hydrogène ou un radical méthyle ; et

$R_2$ représente :

un atome d'hydrogène,

un radical acyle de formule R'-CO- dans laquelle

R' a la signification précédemment définie, ou

un radical benzyloxycarbonyle ;

et de leurs sels avec des acides biologiquement compatibles ;

caractérisé en ce que l'on fait réagir un dérivé halogéné de formule générale II :

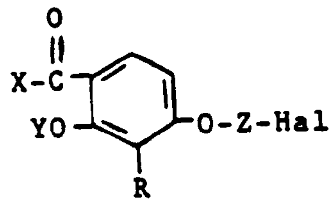

(II)

dans laquelle X, Y, R et Z ont les significations définies ci-dessus et Hal représente un atome d'halogène, avec un dérivé de formule générale III :

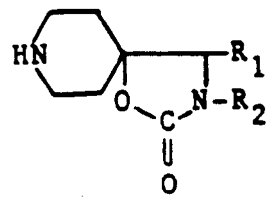

(III)

dans laquelle $R_1$ et $R_2$ sont tels que définis précédemment, et si on le désire, on fait réagir les dérivés ainsi obtenus avec des acides physiologiquement tolérables pour obtenir les sels d'addition correspondants.

2. Le procédé de préparation des dérivés de formule générale Ia :

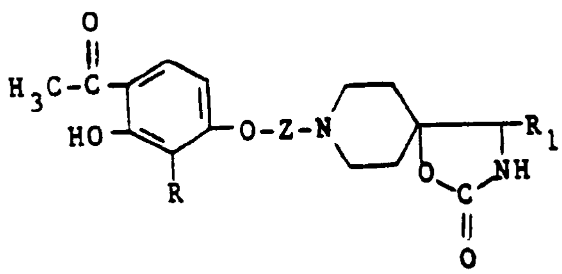

(Ia)

dans laquelle R, Z, et $R_1$ ont les significations définies dans la revendication 1, caractérisé en ce que : l'on effectue la condensation d'un dérivé de formule générale IIa :

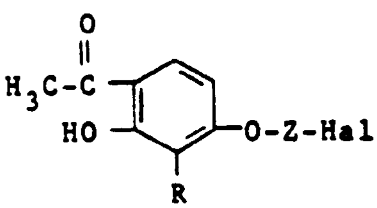

(IIa)

dans laquelle R, Z et Hal sont tels que définis dans la revendication 1, avec un dérivé de formule générale IIIa:

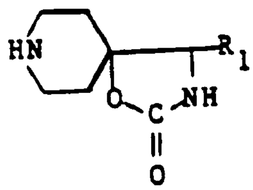

(IIIa)

dans laquelle $R_1$ a la signification énoncée dans la revendication 1.

3. Le procédé de préparation des dérivés de formule générale Ib :

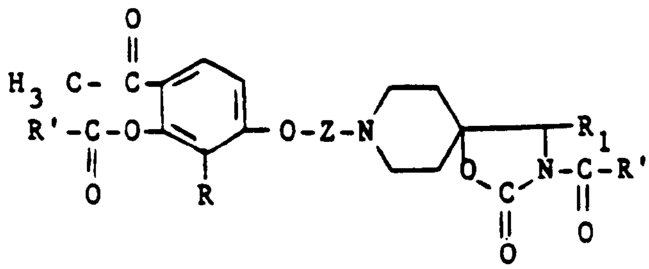

(Ib)

dans laquelle R, R′, Z et $R_1$ ont les significations définies dans la revendication 1, caractérisé en ce que l'on fait réagir un dérivé de formule générale Ia selon la revendication 2, avec la quantité stoechiométrique d'agent acylant de formule :

$$R'COCl \text{ ou } (R'COO)_2O,$$

dans lesquelles R′ a la signification définie dans la revendication 1.

4. Le procédé de préparation des dérivés de formule générale Ic :

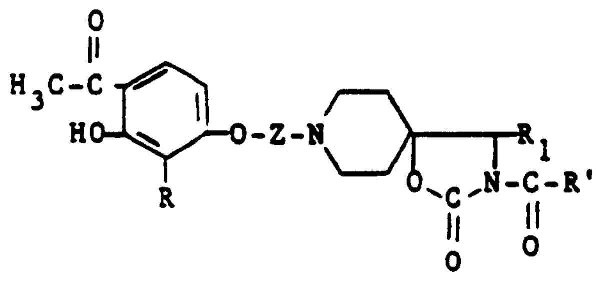

(Ic)

dans laquelle R, Z, R′ et $R_1$ ont les significations définies dans la revendication 1, caractérisé en ce que l'on fait réagir un dérivé de formule générale Ia selon la revendication 2, avec la moitié de la quantité stoechiométrique d'agent acylant R′COCl ou $(R'COO)_2O$ tel que défini dans la revendication 3.

5. Le procédé de préparation des dérivés de formule générale Id, Ie et If ci-après décrites, caractérisé en ce que :

a) l'on fait réagir sur le dérivé Ia défini dans la revendication 2.

un dérivé de formule :

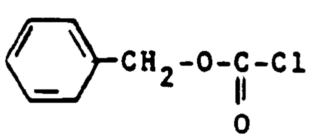

pour obtenir le dérivé de formule générale Id :

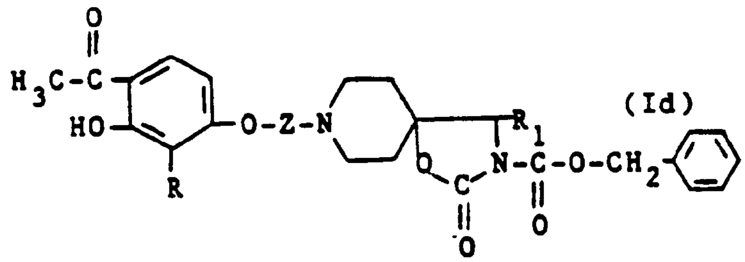

(Id)

dans laquelle R, Z et $R_1$ ont les significations définies dans la revendication 1 ;
b) l'on acyle le dérivé (Id)ainsi obtenu avec un agent acylant R'COCl ou $(R'COO)_2O$ selon la revendication 3, pour obtenir le dérivé de formule générale Ie :

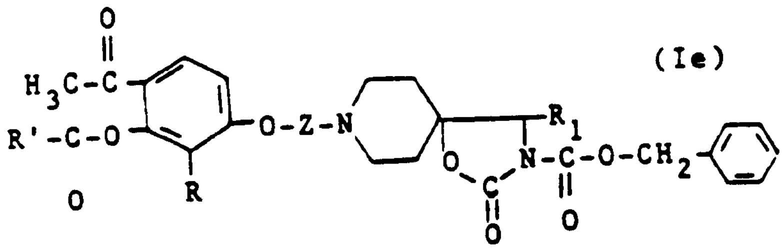

(Ie)

dans laquelle R, R', Z et $R_1$ ont les significations définies dans la revendication 1 ; et
c) l'on hydrogénolyse le dérivé Ie ainsi obtenu au moyen d'hydrogène, en présence de charbon palladié comme catalyseur, pour obtenir le dérivé de formule générale If :

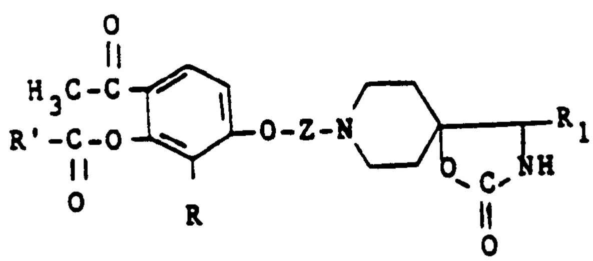

(If)

dans laquelle R, R', Z et $R_1$ ont les significations définies dans la revendication 1.

6. Le procédé de préparation des dérivés de la revendication 1 répondant à la formule générale I dans laquelle X et Y représentent ensemble une chaîne éthénylène ou éthylène substituée par un groupe carboxy, caractérisé en ce que l'on condense le dérivé III selon la revendication 1, sur une forme estérifiée, par un reste méthyle ou éthyle, du dérivé II correspondant selon la revendication 1, puis l'on saponifie le dérivé obtenu au moyen de $NaHCO_3$ pour libérer la fonction carboxy.

**Revendications pour l'Etat contractant suivant : GR**

1. Le procédé de préparation des dérivés du spiro (4,5) décane de formule générale I :

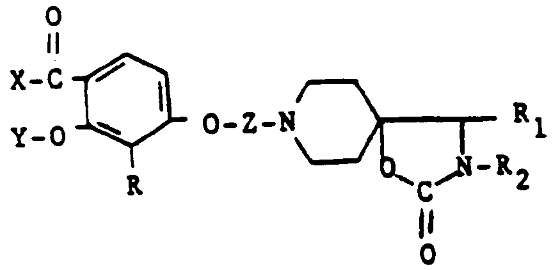

(I)

dans laquelle :

X représente un radical méthyle ; et

Y représente :

un atome d'hydrogène, ou un radical de formule :

R′-CO—

dans laquelle R′ représente :

un radical alkyle ou alkoxy contenant chacun de 1 à 4 atomes de carbone, ou un radical (méthyl ou éthyl)-oxycarbonyle

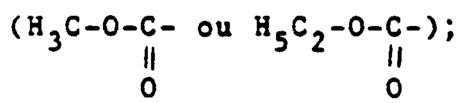

ou

ou X et Y représentent ensemble une chaîne éthénylène de formule

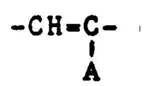

ou éthylène de formule :

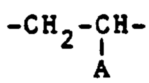

dans lesquelles A représente un atome d'hydrogène, un radical méthyle ou un groupe carboxy, méthoxycarbonyle ou éthoxycarbonyle.

R représente un radical alkyle contenant de 1 à 7 atomes de carbone en chaîne droite ou ramifiée, comportant éventuellement une double liaison ;

Z est une chaîne polyméthylénique $(CH_2)_n$ n étant un nombre entier de 2 à 7, éventuellement ramifiée ou porteuse d'un radical hydroxy ;

$R_1$ représente un atome d'hydrogène ou un radical méthyle ; et

$R_2$ représente :

un atome d'hydrogène, un radical acyle de formule R′-CO- dans laquelle

R′ a la signification précédemment définie, ou un radical benzyloxycarbonyle ; et de leurs sels avec des acides biologiquement compatibles ;

caractérisé en ce que l'on fait réagir un dérivé halogéné de formule générale II :

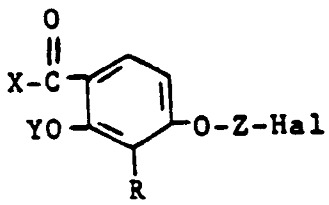

(II)

dans laquelle X, Y, R et Z ont les significations définies ci-dessus et Hal représente un atome d'halogène, avec un dérivé de formule générale III :

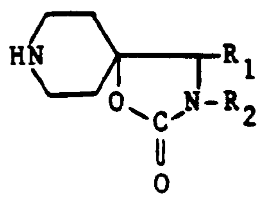

(III)

dans laquelle $R_1$ et $R_2$ sont tels que définis précédemment, et si on le désire, on fait réagir les dérivés ainsi obtenus avec des acides physiologiquement tolérables pour obtenir les sels d'addition correspondants.

2. Le procédé de préparation des dérivés de formule générale Ia :

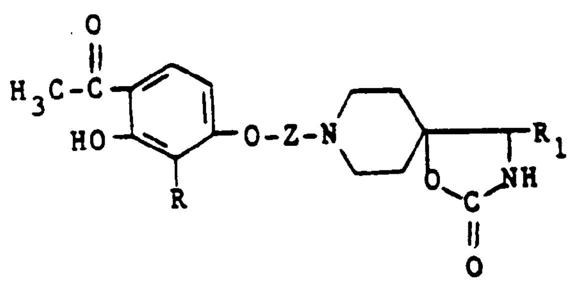

(Ia)

dans laquelle R, Z, et $R_1$ ont les significations définies dans la revendication 1, caractérisé en ce que : l'on effectue la condensation d'un dérivé de formule générale IIa :

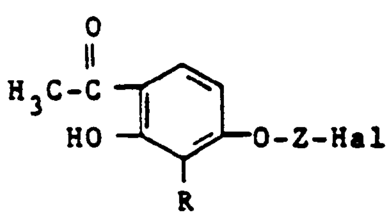

(IIa)

dans laquelle R, Z et Hal sont tels que définis dans la revendication 1, avec un dérivé de formule générale IIIa:

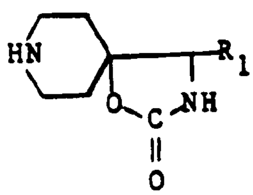

(IIIa)

dans laquelle $R_1$ a la signification énoncée dans la revendication 1.

3. Le procédé de préparation des dérivés de formule générale Ib :

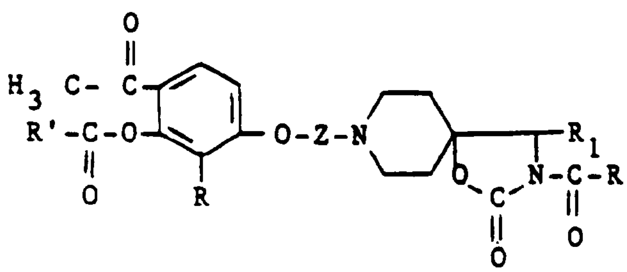

(Ib)

dans laquelle R, R′, Z et $R_1$ ont les significations définies dans la revendication 1, caractérisé en ce que l'on fait réagir un dérivé de formule générale Ia selon la revendication 2, avec la quantité stoechiométrique d'agent acylant de formule :

$$R'COCl \text{ ou } (R'COO)_2O,$$

dans lesquelles R′ a la signification définie dans la revendication 1.

4. Le procédé de préparation des dérivés de formule générale Ic :

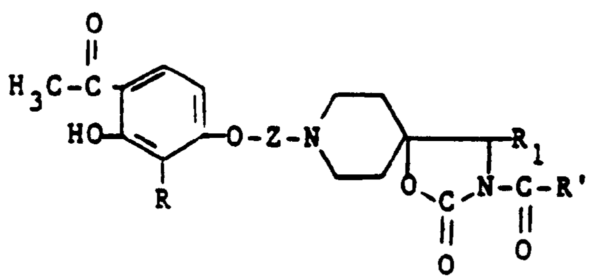

(Ic)

dans laquelle R, Z, R′ et $R_1$ ont les significations définies dans la revendication 1, caractérisé en ce que l'on fait réagir un dérivé de formule générale Ia selon la revendication 2, avec la moitié de la quantité stoechiométrique d'agent acylant R′COCl ou $(R'COO)_2O$ tel que défini dans la revendication 3.

5. Le procédé de préparation des dérivés de formule générale Id, Ie et If ci-après décrites, caractérisé en ce que :

a) l'on fait réagir sur le dérivé Ia défini dans la revendication 2.

un dérivé de formule :

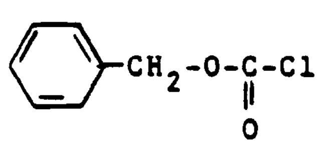

pour obtenir le dérivé de formule générale Id :

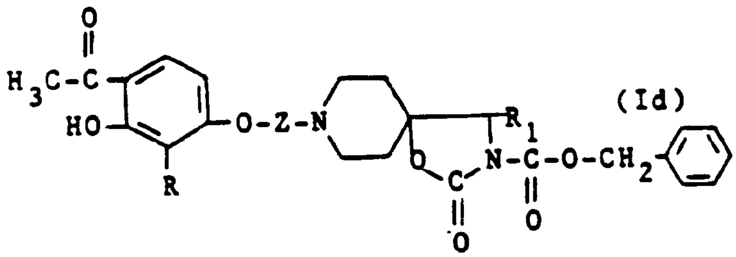

(Id)

dans laquelle R, Z et $R_1$ ont les significations définies dans la revendication 1 ;
b) l'on acyle le dérivé (Id) ainsi obtenu avec un agent acylant R'COCl ou $(R'COO)_2O$ selon la revendication 3, pour obtenir le dérivé de formule générale Ie :

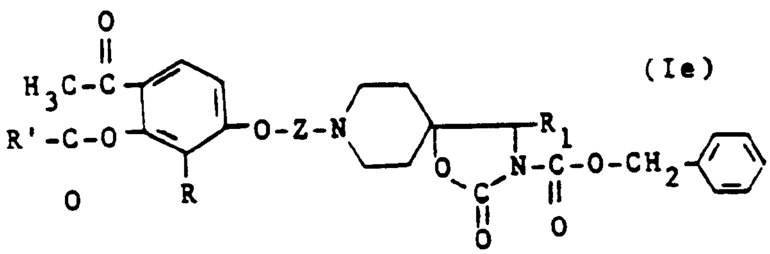

(Ie)

dans laquelle R, R', Z et $R_1$ ont les significations définies dans la revendication 1 ; et
c) l'on hydrogénolyse le dérivé Ie ainsi obtenu au moyen d'hydrogène, en présence de charbon palladié comme catalyseur, pour obtenir le dérivé de formule générale If :

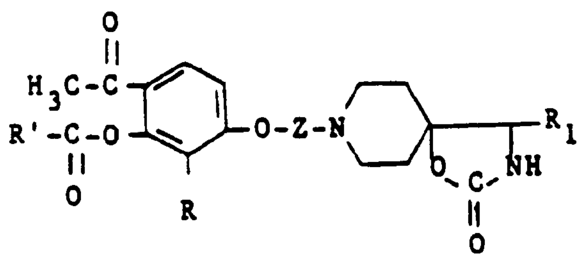

(If)

dans laquelle R, R', Z et $R_1$ ont les significations définies dans la revendication 1.

6. Le procédé de préparation des dérivés de la revendication 1 répondant à la formule générale I dans laquelle X et Y représentent ensemble une chaîne éthénylène ou ethylène substituée par un groupe carboxy, caractérisé en ce que l'on condense le dérivé III selon la revendication 1, sur une forme estérifiée, par un reste méthyle ou éthyle, du dérivé II correspondant selon la revendication 1, puis l'on saponifie le dérivé obtenu au moyen de $NaHCO_3$ pour libérer la fonction carboxy.

**Patentansprüche**

**Patentansprüche für folgende Vertragsstaaten : AT, BE, CH, DE, FR, GB, IT, LI, LU, NL, SE**

1. Spiro[4,5]decan-Derivate der allgemeinen Formel I

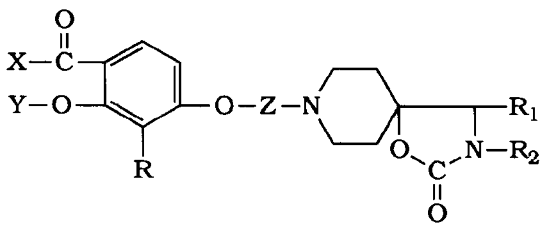

(I)

in der

X eine Methylgruppe ; und

Y ein Wasserstoffatom oder eine Gruppe der Formel

R'CO—

in der R' eine Alkyl- oder Alkoxygruppe mit 1 bis 4 Kohlenstoffatomen darstellt, oder eine (Methyl- oder Ethyl)-oxycarbonylgruppe der Formeln

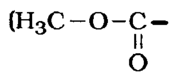 oder 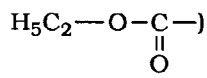

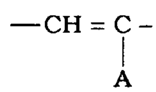

oder X und Y gemeinsam eine Ethenylenkette der Formel oder eine Ethylenkette der Formel

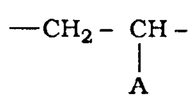

worin A ein Wasserstoffatom, eine Methylgruppe, eine Carboxygruppe, eine Methoxycarbonylgruppe oder eine Ethoxycarbonylgruppe darstellt ;

R eine geradkettige oder verzweigte Alkylgruppe mit 1 bis 7 Kohlenstoffatomen, die gegebenenfalls eine Doppelbindung aufweisen kann ;

Z eine Polymethylenkette der Formel $(CH_2)_n)$, worin n eine ganze Zahl mit einem Wert von 2 bis 7 darstellt, die gegebenenfalls verzweigt sein oder eine Hydroxygruppe tragen kann ;

$R_1$ ein Wasserstoffatom oder eine Methylgruppe ; und

$R_2$ ein Wasserstoffatom, eine Acylgruppe der Formel R'-CO—, worin R' die oben angegebenen Bedeutungen besitzt, oder eine Benzyloxycarbonylgruppe bedeuten

2. Die Salze der Derivate nach Anspruch 1 mit biologisch verträglichen Säuren.

3. 8-[3-(2-Propyl-3-hydroxy-4-acetyl-phenoxy)-propyl]-1 -oxa-2-oxo-3,8diaza-spiro[4,5]decan.

4. 8-[5-(2-Propyl-3-hydroxy-4-acetyl-phenoxy)-pentyl]-1-oxa-2-oxo-3,8-diaza-spiro[4,5]decan.

5. 8-[6-(2-Propyl-3-hydroxy-4-acetyl-phenoxy)-hexyl]-1-oxa-2-oxo-3,8-diaza-spiro[4,5]decan.

6. 8-[3-(2-Propyl-3-acetoxy-4-acetyl-phenoxy)-propyl]-1-oxa-2-oxo-3,8-diaza-3-acetyl-spiro[4,5]decan.

7. 8-[3-(2-Allyl-3-acetoxy-4-acetyl-phenoxy)-propyl]-1-oxa-2-oxo-3,8-diaza-3-acetyl-spiro[4,5]decan.

8. 8-[3-(2-Propyl-3-acetoxy-4-acetyl-phenoxy)-propyl]-1-oxa-2-oxo-3,8-diaza-spiro[4,5]decan.

9. Verfahren zur Herstellung der Derivate nach Anspruch 1, **dadurch gekennzeichnet**, daß man ein Halogenderivat der allgemeinen Formel II

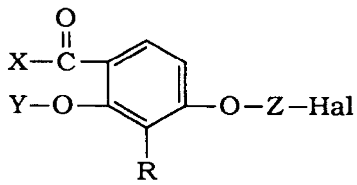

(II)

in der X, Y, R und Z die in Anspruch 1 angegebenen Bedeutungen besitzen und Hal ein Halogellatom darstellt, mit einem Derivat der allgemeinen Formel III

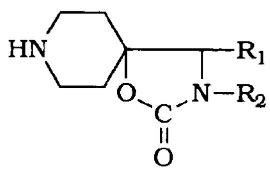

(III)

in der $R_1$ und $R_2$ die in Anspruch 1 angegebenen Bedeutungen besitzen, umsetzt.

10. Verfahren zur Herstellung der Derivate der allgemeinen Formel Ia

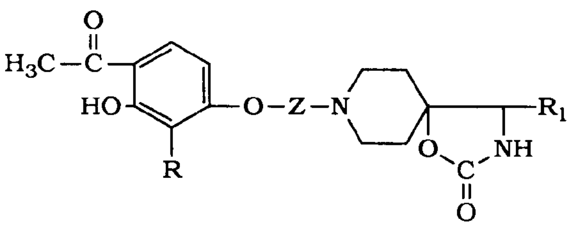

(Ia)

in der R, Z und $R_1$ die in Anspruch 1 angegebenen Bedeutungen besitzen, **dadurch gekennzeichnet,** daß man ein Derivat der allgemeinen Formel IIa

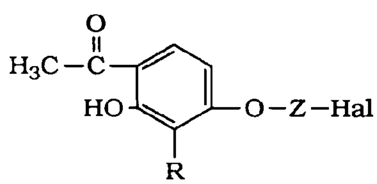

(IIa)

in der R, Z und Hal die in Anspruch 9 angegebenen Bedeutungen besitzen, mit einem Derivat der allgemeinen Formel IIIa

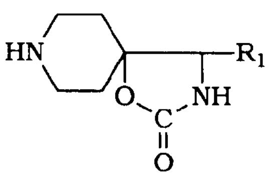

(IIIa)

in der $R_1$ die in Anspruch 9 angegebenen Bedeutungen besitzt, kondensiert.

11. Verfahren zur Herstellung der Derivate der allgemeinen Formel Ib

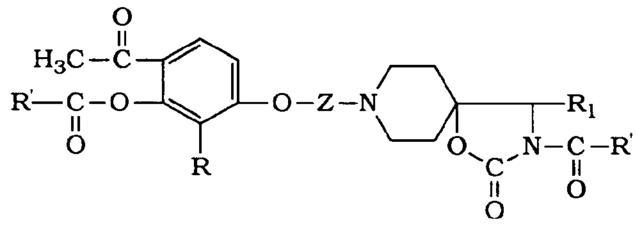

(Ib)

in der R, R′, Z und $R_1$ die in Anspruch 1 angegebenen Bedeutungen besitzen, **dadurch gekennzeichnet,** daß man ein Derivat der allgemeinen Formel Ia nach Anspruch 10 mit der stöchiometrischen Menge eines Acylierungsmittels der allgemeinen Formel

$$R'COCl \text{ oder } (R'COO)_2O,$$

worin R' die in Anspruch 1 angegebenen Bedeutungen besitzt, umsetzt.

12. Verfahren zur Herstellung der Derivate der allgemeinen Formel Ic

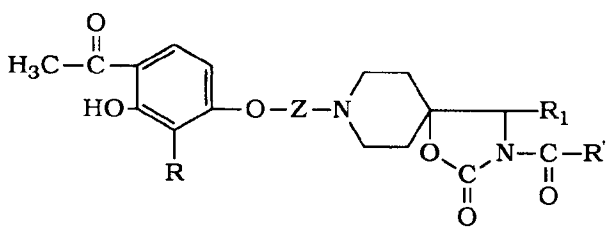

(Ic)

in der R, Z, R′ und $R_1$ die In Anspruch 1 angegebenen Bedeutungen besitzen, **dadurch gekennzeichnet**, daß man ein Derivat der allgemeinen Formel Ia nach Anspruch 10 mit der Hälfte der stöchiometrischen Menge des Acylierungsmlttels der Formel R′COCl oder $(R'COO)_2O$, wie es in Anspruch 11 definiert ist, umsetzt.

13. Verfahren zur Herstellung der nachstehend definierten Derivate der allgemeinen Formeln Id, Ie und If, **dadurch gekennzeichnet,** daß man

a) das in Anspruch 10 definierte Derivat der allgemeinen Formel Ia mit einem Derivat der allgemeinen Formel

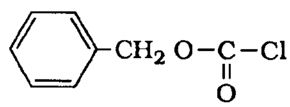

zur Bildung eines Derivats der allgemeinen Formel Id

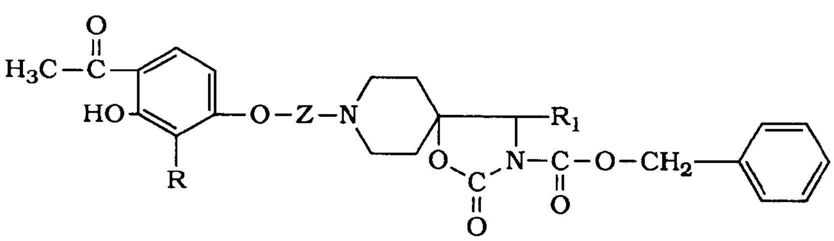

(Id)

worin R, Z und $R_1$ die in Anspruch 1 angegebenen Bedeutungen besitzen, umsetzt ;

b) das in dieser Weise erhaltene Derivat der allgemeinen Formel Id mit einem Acylierungsmittel der Formel R′COCl oder $(R'COO)_2O$ nach der Verfahrensweise des Anspruchs 11 acyliert zur Bildung eines Derivats der allgemeinen Formel Ie

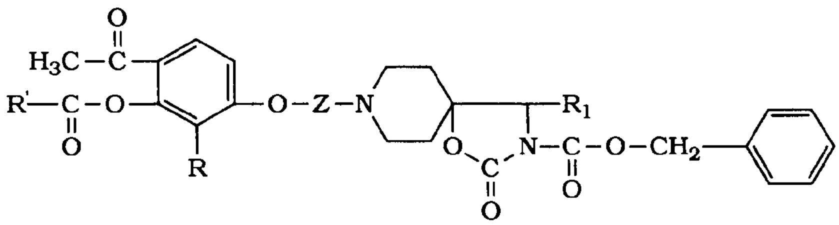

(Ie)

in der R, R′, Z und $R_1$ die in Anspruch 1 angegebenen Bedeutungen besitzen ; und

c) das in dieser Weise erhaltene Derivat der allgemeinen Formel Ie mit Wasserstoff in Gegenwart von Palladium-auf-Kohlenstoff als Katalysator hydrogenolysiert zur Bildung eines Derivats der allgemeinen Formel If

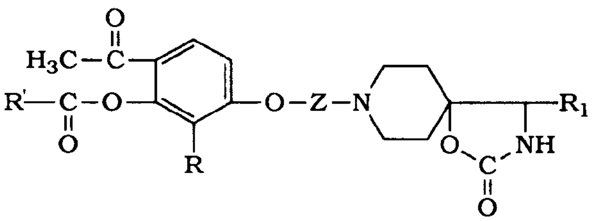

(If)

in der R, R′, Z und $R_1$ die in Anspruch 1 angegebenen Bedeutungen besitzen.

14. Verfahren zur Herstellung der Derivate nach Anspruch 1 der allgemeinen Formel I, in der X und Y gemeinsam eine durch eine Carboxygruppe substituierte Ethenylen- oder Ethylenkette bedeuten, **dadurch gekennzeichnet,** daß man das in Anspruch 9 definierte Derivat der allgemeinen Formel III mit einem in Anspruch 9 definierten Derivat der allgemeinen Formel II in einer durch eine Methylgruppe oder eine Ethylgruppe veresterten Form kondensiert und anschließend das erhaltene Derivat mit $NaHCO_3$ verseift zur Freisetzung der Carboxygruppe.

15. Pharmazeutische Zubereitung enthaltend als Wirkstoff ein Derivat nach einem der Ansprüche 1 bis 8 zusammen mit pharmazeutisch geeigneten Trägermaterialien.

16. Pharmazeutische Zubereitung nach Anspruch 15 in einer Form, die insbesondere zur Behandlung von Asthma, allergischen Phänomenen, chronischen obstruktiven Bronchopathien, Lungenarterienhypertensionen und Entzündungszuständen der oberen Atemwege geeignet ist.

**Patentansprüche für folgende Vertragsstaaten : ES**

1. Verfahren zur Herstellung von Spiro[4,5]decan-Derivaten der allgemeinen Formel I

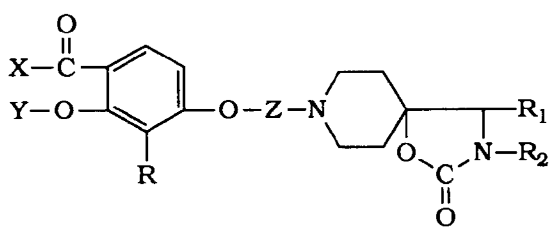

(I)

in der

X eine Methylgruppe ; und

Y ein Wasserstoffatom oder eine Gruppe der Formel

R′CO—

in der R′ eine Alkyl- oder Alkoxygruppe mit 1 bis 4 Kohlenstoffatomen darstellt, oder eine (Methyl- oder Ethyl)-oxycarbonylgruppe der Formeln

(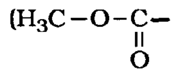 oder 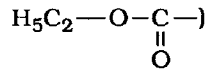)

oder X und Y gemeinsam eine Ethenylenkette der Formel

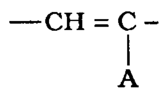

oder eine Ethylenkette der Formel

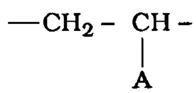

worin A ein Wasserstoffatom, eine Methylgruppe, eine Carboxygruppe, eine Methoxycarbonylgruppe oder eine Ethoxycarbonylgruppe darstellt ;
R eine geradkettige oder verzweigte Alkylgruppe mit 1 bis 7 Kohlenstoffatomen, die gegebenenfalls eine Doppelbindung aufwelsen kann ;
Z eine Polymethylenkette der Formel ($(CH_2)n$), worin n eine ganze Zahl mit einem Wert von 2 bis 7 darstellt, die gegebenenfalls verzweigt sein oder eine Hydroxygruppe tragen kann ;
$R_1$ ein Wasserstoffatom oder eine Methylgruppe ; und
$R_2$ ein Wasserstoffatom, eine Acylgruppe der Formel R′-CO—, worin R′ die oben angegebenen Bedeutungen besitzt, oder eine Benzyloxycarbonylgruppe bedeuten, und deren Salzen mit biologisch verträglichen Säuren, **dadurch gekennzeichnet,** daß man ein Halogenderivat der allgemeinen Formel II

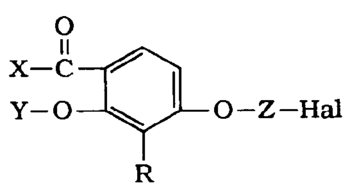 (II)

in der X, Y, R und Z die oben angegebenen Bedeutungen besitzen und Hal ein Halogenatom darstellt, mit einem Derivat der allgemeinen Formel III

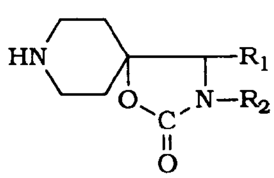 (III)

in der $R_1$ und $R_2$ die oben angegebenen Bedeutungen besitzen, umsetzt.
2. Verfahren zur Herstellung der Derivate der allgemeinen Formel Ia

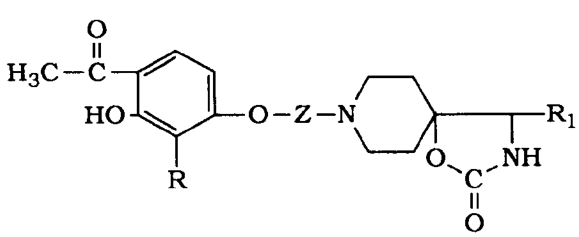 (Ia)

in der R, Z und $R_1$ die in Anspruch 1 angegebenen Bedeutungen besitzen, **dadurch gekennzeichnet,** daß man ein Derivat der allgemeinen Formel IIa

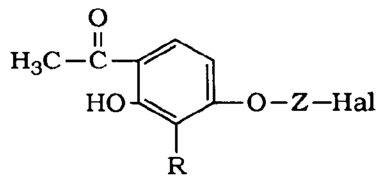

(IIa)

in der R, Z und Hal die In Anspruch 1 angegebenen Bedeutungen besitzen, mit einem Derivat der allgemeinen Formel IIIa

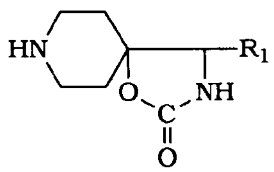

(IIIa)

in der $R_1$ die in Anspruch 1 angegebenen Bedeutungen besitzt, kondensiert.

3. Verfahren zur Herstellung der Derivate der allgemeinen Formel Ib

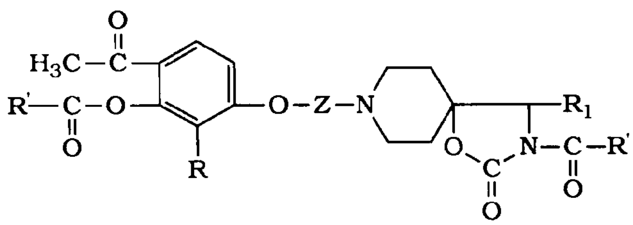

(Ib)

in der R, R′, Z und $R_1$ die in Anspruch 1 angegebenen Bedeutungen besitzen, **dadurch gekennzeichnet**, daß man ein Derivat der allgemeinen Formel Ia nach Anspruch 2 mit der stöchiometrischen Menge eines Acylierungsmittels der allgemeinen Formel

$$R'COCl \text{ oder } (R'COO)_2O,$$

worin R′ die in Anspruch 1 angegebenen Bedeutungen besitzt, umsetzt.

4. Verfahren zur Herstellung der Derivate der allgemeinen Formel Ic

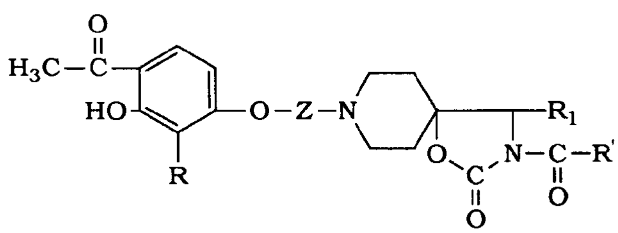

(Ic)

in der R, Z, R′ und $R_1$ die in Anspruch 1 angegebenen Bedeutungen besitzen, **dadurch gekennzeichnet,** daß man ein Derivat der allgemeinen Formel Ia nach Anspruch 2 mit der Hälfte der stöchiometrischen Menge des Acylierungsmittels der Formel R′COCl oder $(R'COO)_2O$, wie es in Anspruch 3 definiert ist, umsetzt.

5. Verfahren zur Herstellung der nachstehend definierten Derivate der allgemeinen Formeln Id, Ie und If, **dadurch gekennzeichnet**, daß man

a) das in Anspruch 2 definierte Derivat der allgemeinen Formel Ia mit einem Derivat der allgemeinen Formel

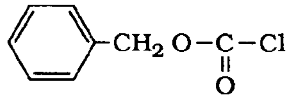

zur Bildung eines Derivats der allgemeinen Formel Id

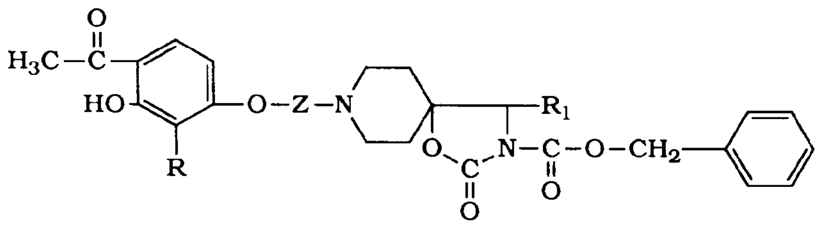

(Id)

worin R, Z und $R_1$ die in Anspruch 1 angegebenen Bedeutungen besitzen, umsetzt ;

b) das in dieser Weise erhaltene Derivat der allgemeinen Formel Id mit einem Acylierungsmittel der Formel R'COCl oder $(R'COO)_2O$ nach der Verfahrensweise des Anspruchs 3 acyliert zur Bildung eines Derivats der allgemeinen Formel Ie

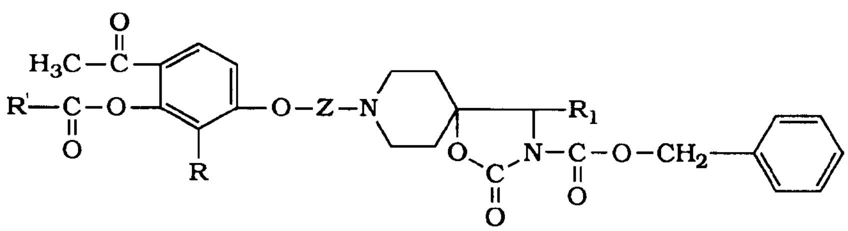

(Ie)

in der R, R', Z und $R_1$ die in Anspruch 1 angegebenen Bedeutungen besitzen ; und

c) das in dieser Weise erhaltene Derivat der allgemeinen Formel Ie mit Wasserstoff in Gegenwart von Palladium-auf-Kohlenstoff als Katalysator hydrogenolysiert zur Bildung eines Derivats der allgemeinen Formel If

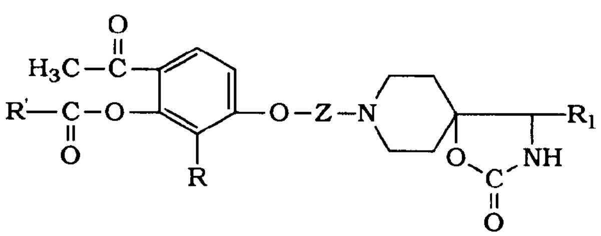

(If)

in der R, R', Z und $R_1$ die in Anspruch 1 angegebenen Bedeutungen besitzen.

6. Verfahren zur Herstellung der Derivate nach Anspruch 1 der allgemeinen Formel I, in der X und Y gemeinsam eine durch eine Carboxygruppe substituierte Ethenylen- oder Ethylenkette bedeuten, **dadurch gekennzeichnet,** daß man das in Anspruch 9 definierte Derivat der allgemeinen Formel III mit einem in Anspruch 9 definierten Derivat der allgemeinen Formel II in einer durch eine Methylgruppe oder eine Ethylgruppe veresterten Form kondensiert und anschließend das erhaltene Derivat mit $NaHCO_3$ verseift zur Freisetzung der Carboxygruppe.

**Patentansprüche für folgende Vertragsstaaten : GR**

1. Verfahren zur Herstellung von Spiro[4,5]decan-Derivaten der allgemeinen Formel I

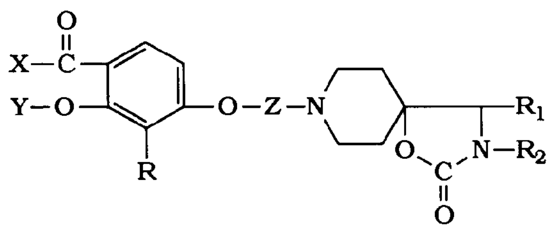

(I)

in der

X eine Methylgruppe ; und

Y ein Wasserstoffatom oder eine Gruppe der Formel

R'CO—

in der R' eine Alkyl- oder Alkoxygruppe mit 1 bis 4 Kohlenstoffatomen darstellt, oder eine (Methyl- oder Ethyl)-oxycarbonylgruppe der Formeln

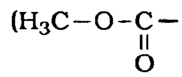 oder 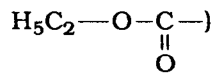

oder X und Y gemeinsam eine Ethenylenkette der Formel

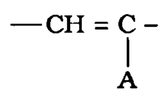

oder eine Ethylenkette der Formel

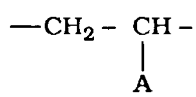

worin A ein Wasserstoffatom, eine Methylgruppe, eine Carboxygruppe, eine Methoxycarbonylgruppe oder eine Ethoxycarbonylgruppe darstellt,

R eine geradkettige oder verzweigte Alkylgruppe mit 1 bis 7 Kohlenstoffatomen, die gegebenenfalls eine Doppelbindung aufweisen kann ;

Z eine Polymethylenkette der Formel $(CH_2)n)$, worin n eine ganze Zahl mit einem Wert von 2 bis 7 darstellt, die gegebenenfalls verzweigt sein oder eine Hydroxygruppe tragen kann ;

$R_1$ ein Wasserstoffatom oder eine Methylgruppe ; und

$R_2$ ein Wasserstoffatom, eine Acylgruppe der Formel R'-CO—, worin R' die oben angegebenen Bedeutungen besitzt, oder eine Benzyloxycarbonylgruppe bedeuten, und deren Salzen mit biologisch verträglichen Säuren, **dadurch gekennzeichnet,** daß man ein Halogenderivat der allgemeinen Formel II

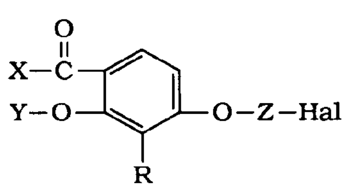

(II)

in der X, Y, R und Z die oben angegebenen Bedeutungen besitzen und Hal ein Halogenatom darstellt, mit einem Derivat der allgemeinen Formel III

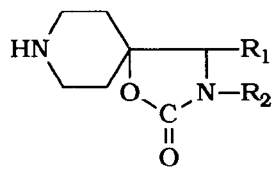

(III)

in der $R_1$ und $R_2$ die oben angegebenen Bedeutungen besitzen, umsetzt.

2. Verfahren zur Herstellung der Derivate der allgemeinen Formel Ia

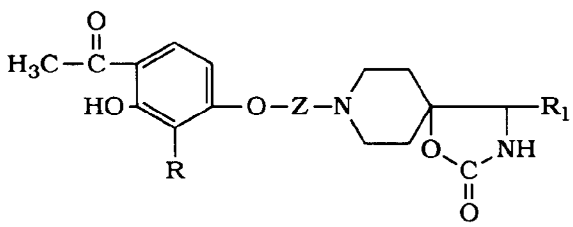

(Ia)

in der R, Z und $R_1$ die in Anspruch 1 angegebenen Bedeutungen besitzen, **dadurch gekennzeichnet,** daß man ein Derivat der allgemeinen Formel IIa

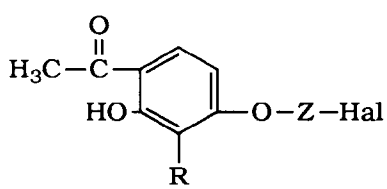

(IIa)

in der R, Z und Hal die in Anspruch 1 angegebenen Bedeutungen besitzen, mit einem Derivat der allgemeinen Formel IIIa

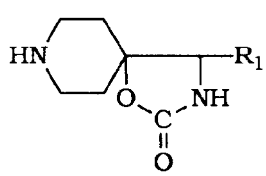

(IIIa)

in der $R_1$ die in Anspruch 1 angegebenen Bedeutungen besitzt, kondensiert.

3. Verfahren zur Herstellung der Derivate der allgemeinen Formel Ib

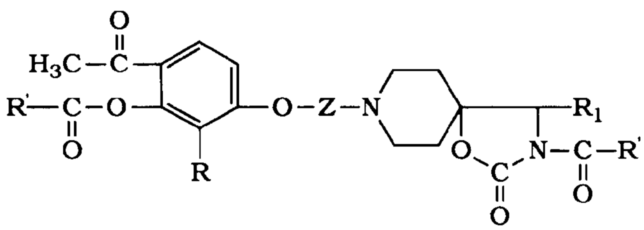

(Ib)

in der R, R′, Z und $R_1$ die in Anspruch 1 angegebenen Bedeutungen besitzen, **dadurch gekennzeichnet,** daß man ein Derivat der allgemeinen Formel Ia nach Anspruch 2 mit der stöchiometrischen Menge eines Acylierungsmittels der allgemeinen Formel

R′COCL oder $(R'COO)_2O$,

worin R′ die in Anspruch 1 angegebenen Bedeutungen besitzt, umsetzt.

4. Verfahren zur Herstellung der Derivate der allgemeinen Formel Ic

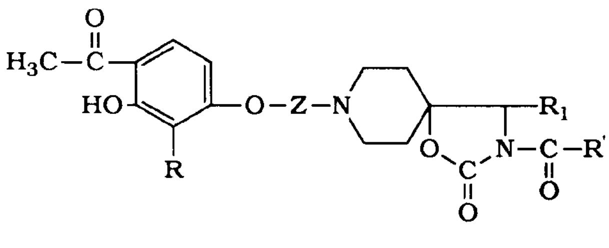

(Ic)

in der R, Z, R′ und $R_1$ die in Anspruch 1 angegebenen Bedeutungen besitzen, **dadurch gekennzeichnet**, daß man ein Derivat der allgemeinen Formel Ia nach Anspruch 2 mit der Hälfte der stöchiometrischen Menge des Acylierungsmittels der Formel R′COCl oder $(R'COO)_2O$, wie es in Anspruch 3 definiert ist, umsetzt.

5. Verfahren zur Herstellung der nachstehend definierten Derivate der allgemeinen Formeln Id, Ie und If, dadurch gekennzelchnet, daß man

a) das in Anspruch 2 definierte Derivat der allgemeinen Formel Ia mit einem Derivat der allgemeinen Formel

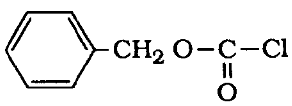

zur Bildung eines Derivats der allgemeinen Formel Id

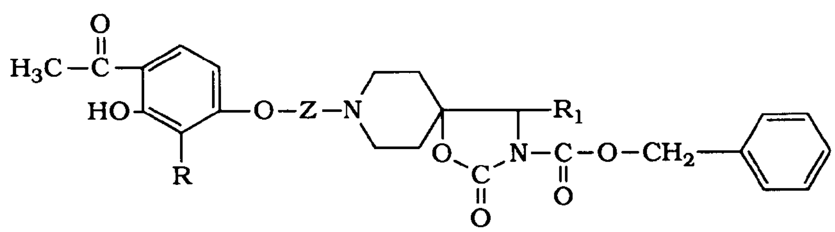

(Id)

worin R, Z und $R_1$ die in Anspruch 1 angegebenen Bedeutungen besitzen, umsetzt ;

b) das in dieser Weise erhaltene Derivat der allgemeinen Formel Id mit einem Acylierungsmittel der Formel R′COCl oder $(R'COO)_2O$ nach der Verfahrensweise des Anspruchs 3 acyliert zur Bildung eines Derivats der allgemeinen Formel Ie

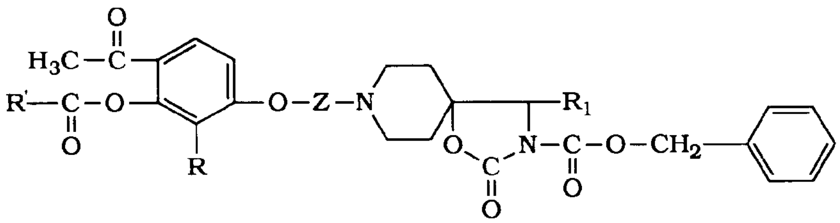

(Ie)

in der R, R′, Z und $R_1$ die in Anspruch 1 angegebenen Bedeutungen besitzen ; und

c) das in dieser Weise erhaltene Derivat der allgemeinen Formel Ie mit Wasserstoff in Gegenwart von Palladium-auf-Kohlenstoff als Katalysator hydrogenolysiert zur Bildung eines Derivats der allgemeinen Formel If

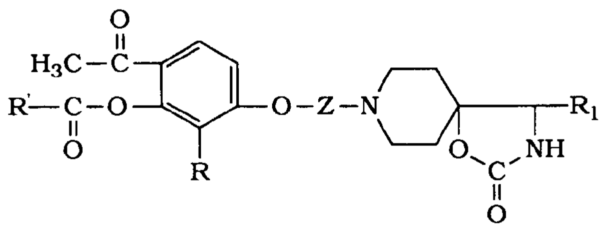

(If)

in der R, R′, Z und $R_1$ die in Anspruch 1 angegebenen Bedeutungen besitzen.

6. Verfahren zur Herstellung der Derivate nach Anspruch 1 der allgemeinen Formel I, in der X und Y gemeinsam eine durch eine Carboxygruppe substituierte Ethenylen- oder Ethylenkette bedeuten, **dadurch gekennzeichnet**, daß man das in Anspruch 9 definierte Derivat der allgemeinen Formel III mit einem in Anspruch 9 definierten Derivat der allgemeinen Formel II in einer durch eine Methylgruppe oder eine Ethylgruppe veresterten Form kondensiert und anschließend das erhaltene Derivat mit $NaHCO_3$ verseift zur Freisetzung der Carboxygruppe.

## Claims

### Claims for the following Contracting States : AT, BE, CH, DE, FR, GB, IT, LI, LU, NL, SE

1. Spiro[4.5]decane derivatives of the general formula I :

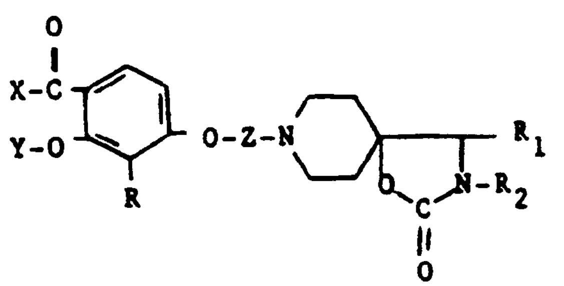

(I)

in which :

X represents a methyl radical ; and

Y represents :

a hydrogen atom, or a radical of the formula :

R′-CO—

in which R′ represents :

an alkyl or alkoxy radical each containing from 1 to 4 carbon atoms,

or a (methyl or ethyl)-oxycarbonyl radical

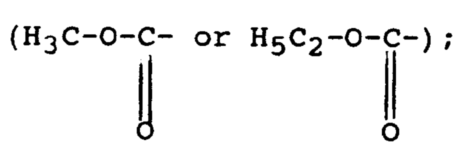

or X and Y together represent an ethenylene chain of the formula :

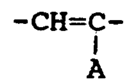

or an ethylene chain of the formula :

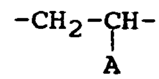

in each of which A represents a hydrogen atom, a methyl radical or a carboxy, methoxycarbonyl or ethoxycarbonyl group ;

R represents a straight-chain or branched alkyl radical containing from 1 to 7 carbon atoms, optionally containing a double bond ;

Z is a polymethylene chain $(CH_2)_n$, n being an integer from 2 to 7, optionally branched or carrying a hydroxy radical ;

$R_1$ represents a hydrogen atom or a methyl radical ; and

$R_2$ represents :

a hydrogen atom, an acyl radical of the formula R'-CO— in which R' has the meaning defined above, or a benzyloxycarbonyl radical.

2. The salts of the derivatives of claim 1 with biologically compatible acids.

3. 8-[3-(2-propyl-3-hydroxy-4-acetylphenoxy)propyl]-1-oxa-2-oxo-3,8-diaza-spiro[4.5]decane.

4. 8-[5-(2-propyl-3-hydroxy-4-acetylphenoxy)pentyl]-1-oxa-2-oxo-3,8-diaza-spiro[4.5]decane.

5. 8-[6-(2-propyl-3-hydroxy-4-acetylphenoxy)hexyl]-1-oxa-2-oxo-3,8-diaza-spiro[4.5]decane.

6. 8-[3-(2-propyl-3-acetoxy-4-acetylphenoxy)propyl]-1-oxa-2-oxo-3,8-diaza-3-acetyl-spiro[4.5]decane.

7. 8-[3-(2-allyl-3-acetoxy-4-acetylphenoxy)propyl]-1-oxa-2-oxo-3,8-diaza-3-acetyl-spiro[4.5]decane.

8. 8-[3-(2-propyl-3-acetoxy-4-acetylphenoxy)propyl]-1-oxa-2-oxo-3,8-diaza-spiro[4.5]decane.

9. Process for the preparation of the derivatives of claim 1, characterised in that a halogenated derivative of the general formula II :

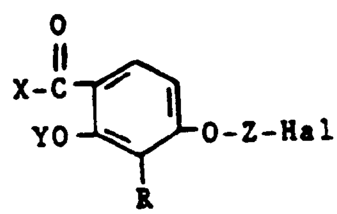

(II)

in which X, Y, R and Z have the meanings defined in claim 1 and Hal represents a halogen atom, is reacted with a derivative of the general formula III :

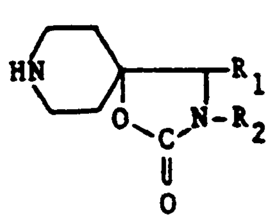

(III)

in which $R_1$ and $R_2$ are as defined in claim 1.

10. Process for the preparation of the derivatives of the general formula Ia :

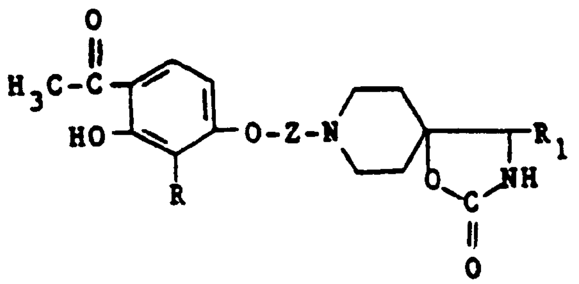

(Ia)

in which R, Z and $R_1$ have the meanings defined in claim 1, characterised in that : a derivative of the general formula IIa :

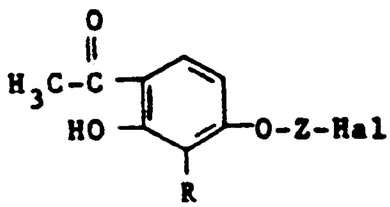

(IIa)

in which R, Z and Hal are as defined in claim 9, is condensed with a derivative of the general formula IIIa :

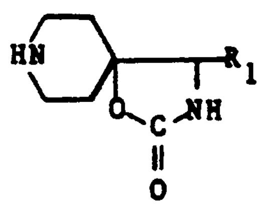

(IIIa)

in which $R_1$ has the meaning given in claim 9.

11. Process for the preparation of the derivatives of the general formula Ib :

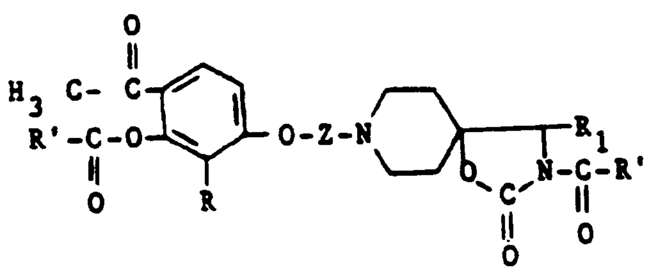

(Ib)

in which R, R′, Z and $R_1$ have the meanings defined in claim 1, characterised in that a derivative of the general formula Ia according to claim 10 is reacted with the stoichiometric amount of acylating agent of the formula :

$$R'COCl \text{ or } (R'COO)_2O$$

in each of which R′ has the meaning defined in claim 1.

12. Process for the preparation of the derivatives of the general formula Ic :

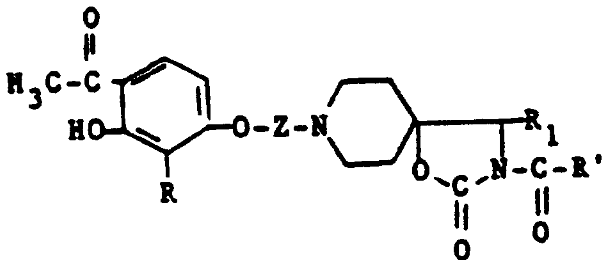

(Ic)

in which R, Z, R′ and $R_1$ have the meanings defined in claim 1, characterised in that a derivative of the general formula Ia according to claim 10 is reacted with half the stoichiometric amount of acylating agent R′COCl or $(R'COO)_2O$ as defined in claim 11.

13. Process for the preparation of the derivatives of the general formulae Id, Ie and If described below, characterised in that :

a) a derivative of the formula :

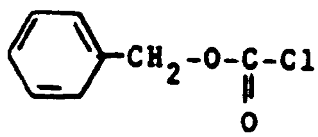

is reacted with derivative Ia defined in claim 10 to obtain the derivative of the general formula Id :

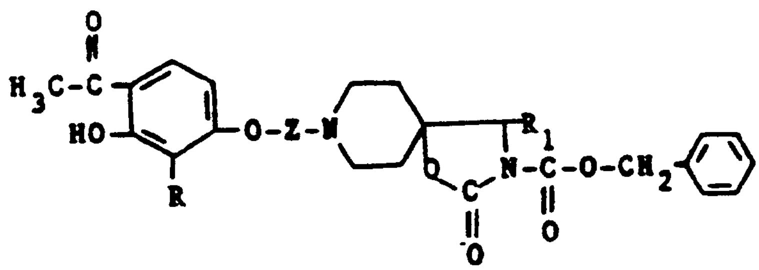

(Id)

in which R, Z and $R_1$ have the meanings defined in claim 1 ;

b) the derivative (Id) so obtained is acylated with an acylating agent R′COCl or $(R'COO)_2O$ according to claim 11, to obtain the derivative of the general formula Ie :

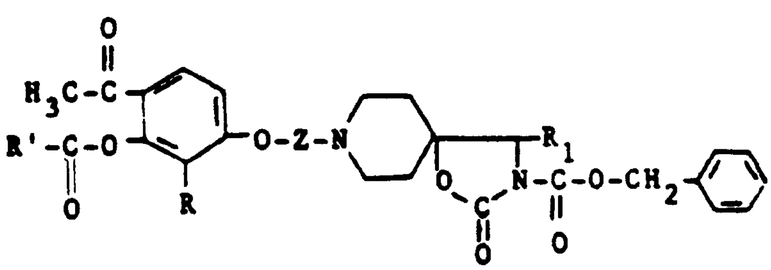

(Ie)

in which R, R′, Z and $R_1$ have the meanings defined in claim 1 ; and

c) the derivative Ie so obtained is subjected to hydrogenolysis using hydrogen, in the presence of palladium/carbon as catalyst, to obtain the derivative of the general formula If :

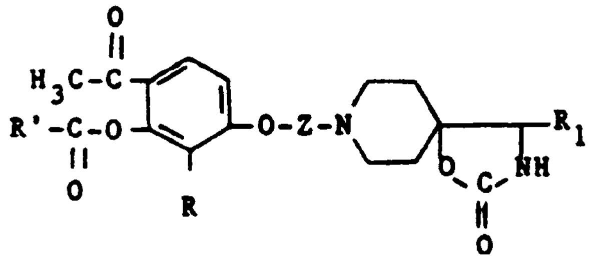

(If)

in which R, R′, Z and $R_1$ have the meanings defined in claim 1.

14. Process for the preparation of the derivatives of claim 1 corresponding to the general formula I in which X and Y together represent an ethenyl or ethylene chain substituted by a carboxy group, characterised in that derivative III according to claim 9 is condensed with a methyl- or ethyl-esterified form of the corresponding derivative II according to claim 9, and then the resulting derivative is hydrolysed using $NaHCO_3$ in order to free the carboxy function.

15. Pharmaceutical compositions containing as active ingredient a derivative according to claims 1 to 8, with suitable pharmaceutical excipients.

16. Pharmaceutical compositions according to claim 15 presented in a form suitable in particular for the treatment of asthma, allergic phenomena, chronic obstructive bronchopathies, pulmonary arterial hypertension and inflammatory diseases of the upper respiratory passages.

**Claims for the following Contracting States : ES**

1. Process for the preparation of spiro[4.5]decane derivatives of the general formula I :

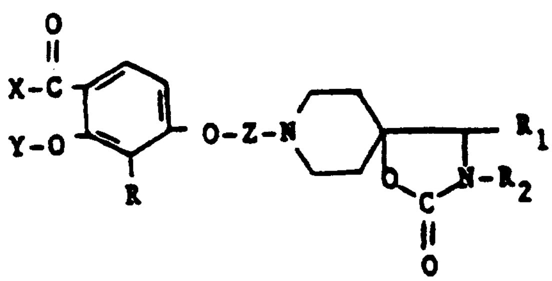

(I)

in which :

X represents a methyl radical ; and

Y represents :

a hydrogen atom, or a radical of the formula :

R′-CO—

in which R′ represents :

an alkyl or alkoxy radical each containing from 1 to 4 carbon atoms, or a (methyl or ethyl)-oxycarbonyl radical

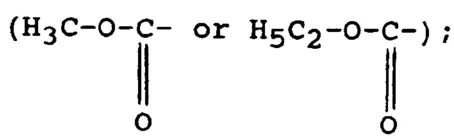

or X and Y together represent an ethenylene chain of the formula :

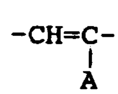

or an ethylene chain of the formula :

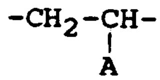

in each of which A represents a hydrogen atom, a methyl radical or a carboxy, methoxycarbonyl or ethoxycarbonyl group ;
R represents a straight-chain or branched alkyl radical containing from 1 to 7 carbon atoms, optionally containing a double bond ;
Z is a polymethylene chain $(CH_2)_n$, n being an integer from 2 to 7, optionally branched or carrying a hydroxy radical ;
$R_1$ represents a hydrogen atom or a methyl radical ; and
$R_2$ represents :
a hydrogen atom, an acyl radical of the formula R'-CO— in which R' has the meaning defined above, or a benzyloxycarbonyl radical ;
and their salts with biologically compatible acids ; characterised in that a halogenated derivative of the general formula II :

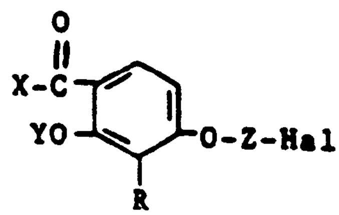

(II)

in which X, Y, R and Z have the meanings defined above and Hal represents a halogen atom, is reacted with a derivative of the general formula III :

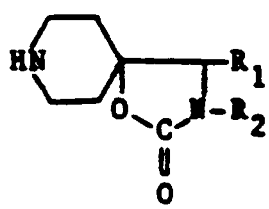

(III)

in which $R_1$ and $R_2$ are as defined above, and, if desired, the derivatives so obtained are reacted with physiologically tolerable acids to obtain the corresponding addition salts.

2. Process for the preparation of the derivatives of the general formula Ia :

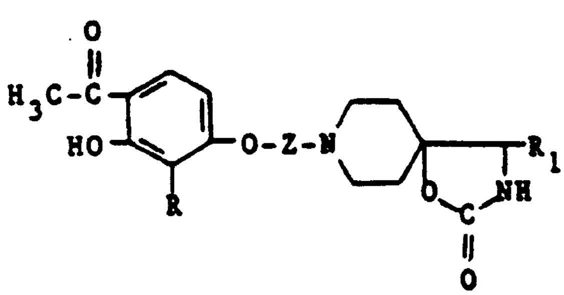

(Ia)

in which R, Z and $R_1$ have the meanings defined in claim I, characterised in that :

a derivative of the general formula IIa :

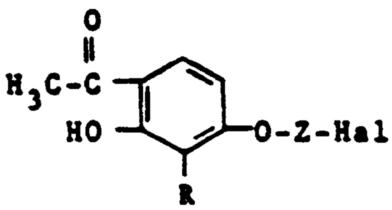

(IIa)

in which R, Z and Hal are as defined in claim 1, is condensed with a derivative of the general formula IIIa :

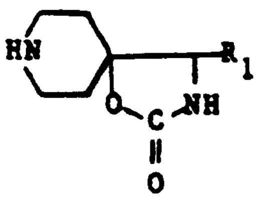

(IIIa)

in which $R_1$ has the meaning given in claim 1.

3. Process for the preparation of the derivatives of the general formula Ib :

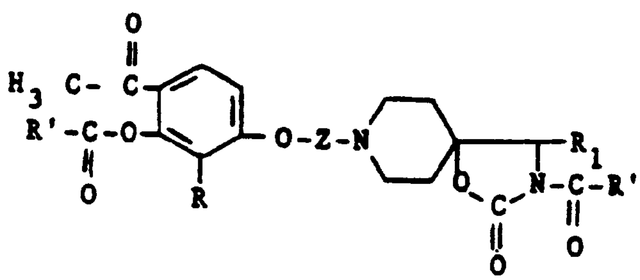

(Ib)

in which R, R′, Z and $R_1$ have the meanings defined in claim 1, characterised in that a derivative of the general formula Ia according to claim 2 is reacted with the stoichiometric amount of acylating agent of the formula :

$$R'COCl \text{ or } (R'COO)_2O$$

in each of which R′ has the meaning defined in claim 1.

4. Process for the preparation of the derivatives of the general formula Ic :

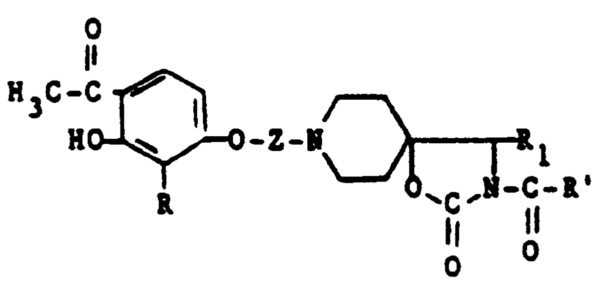

(Ic)

in which R, Z, R′ and $R_1$ have the meanings defined in claim 1, characterised in that a derivative of the general formula Ia according to claim 2 is reacted with half the stoichiometric amount of acylating agent R′COCl or $(R'COO)_2O$ as defined in claim 3.

5. Process for the preparation of the derivatives of the general formulae Id, Ie and If described below, characterised in that :

a) a derivative of the formula :

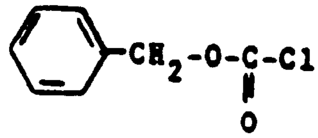

is reacted with derivative Ia defined in claim 2 to obtain the derivative of the general formula Id :

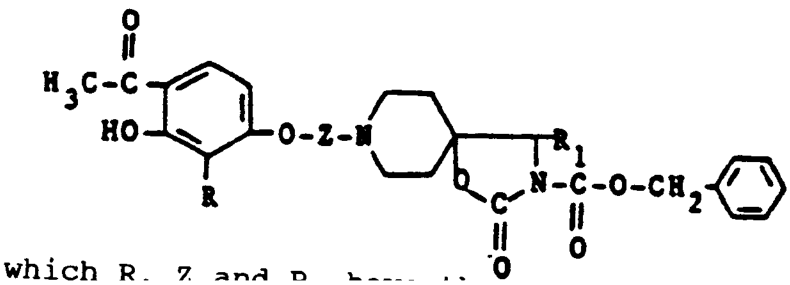

(Id)

in which R, Z and $R_1$ have the meanings defined in claim 1 ;

b) the derivative (Id) so obtained is acylated with an acylating agent R'COCl or $(R'COO)_2O$ according to claim 3, to obtain the derivative of the general formula Ie :

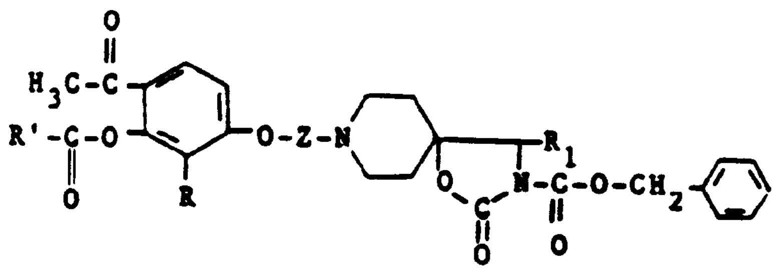

(Ie)

in which R, R', Z and $R_1$ have the meanings defined in claim 1 ; and

c) the derivative Ie so obtained is subjected to hydrogenolysis using hydrogen, in the presence of palladian/carbon as catalyst, to obtain the derivative of the general formula If :

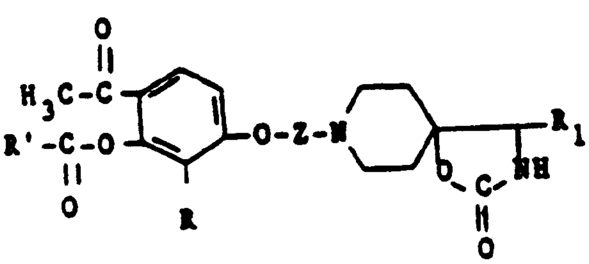

(If)

in which R, R', Z and $R_1$ have the meanings defined in claim 1.

6. Process for the preparation of the derivatives of claim 1 corresponding to the general formula I in which X and Y together represent an ethenylene or ethylene chain substituted by a carboxy group, characterised in that derivative III according to claim 1 is condensed with a methyl- or ethyl-esterified form of the corresponding derivative II according to claim 1, and then the resulting derivative is hydrolysed using $NaHCO_3$ in order to free the carboxy function.

**Claims for the following Contracting States : GR**

1. Process for the preparation of spiro[4.5]decane derivatives of the general formula I :

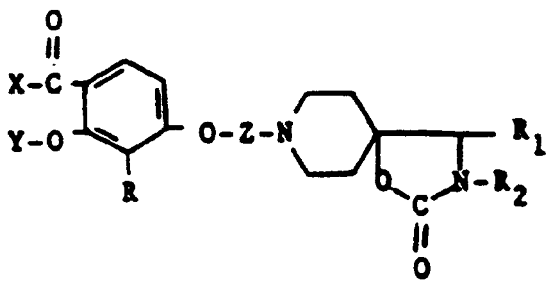

(I)

in which :

X represents a methyl radical ; and

Y represents :

a hydrogen atom, or a radical of the formula :

R'-CO—

in which R' represents :

an alkyl or alkoxy radical each containing from 1 to 4 carbon atoms, or a (methyl or ethyl)-oxycarbonyl radical

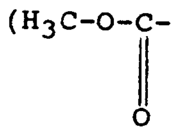

or

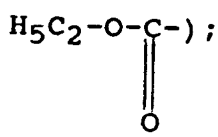

or X and Y together represent an ethenylene chain of the formula :

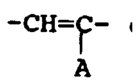

or an ethylene chain of the formula :

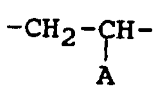

in each of which A represents a hydrogen atom, a methyl radical or a carboxy, methoxycarbonyl or ethoxycarbonyl group ;

R represents a straight-chain or branched alkyl radical containing from 1 to 7 carbon atoms, optionally containing a double bond ;

Z is a polymethylene chain $(CH_2)n$, n being an integer from 2 to 7, optionally branched or carrying a hydroxy radical ;

$R_1$ represents a hydrogen atom or a methyl radical ; and

$R_2$ represents :

a hydrogen atom, an acyl radical of the formula R'-CO— in which R' has the meaning defined above, or a benzyloxycarbonyl radical ;

and their salts with biologically compatible acids ;

characterised in that a halogenated derivative of the general formula II :

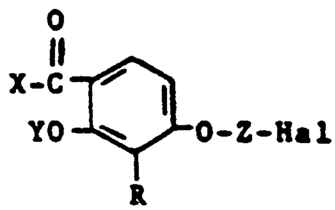

(II)

in which X, Y, R and Z have the meanings defined above and Hal represents a halogen atom, is reacted with a derivative of the general formula III :

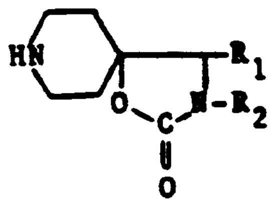

(III)

in which $R_1$ and $R_2$ are as defined above, and, if desired, the derivatives so obtained are reacted with physiologically tolerable acids to obtain the corresponding addition salts.

2. Process for the preparation of the derivatives of the general formula Ia :

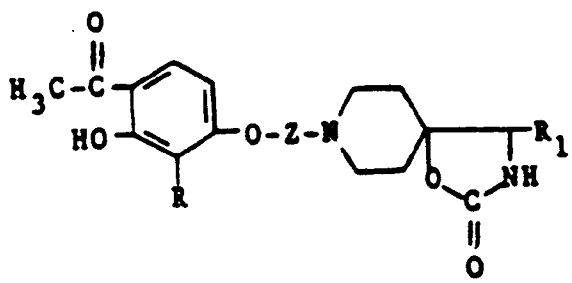

(Ia)

in which R, Z and $R_1$ have the meanings defined in claim 1, characterised in that :
a derivative of the general formula IIa :

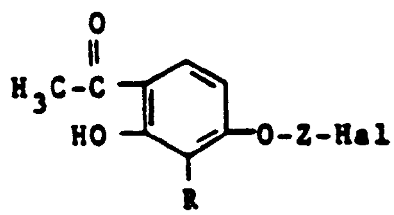

(IIa)

in which R, Z and Hal are as defined in claim 1, is condensed with a derivative of the general formula IIIa :

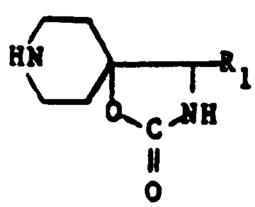

(IIIa)

in which $R_1$ has the meaning given in claim 1.

3. Process for the preparation of the derivatives of the general formula Ib :

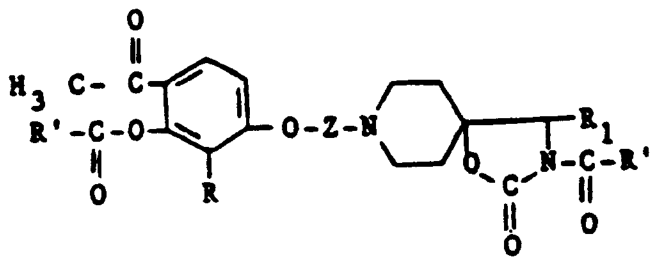

(Ib)

in which R, R′, Z and $R_1$ have the meanings defined in claim 1, characterised in that a derivative of the general formula Ia according to claim 2 is reacted with the stoichiometric amount of acylating agent of the formula :

$$R'COCl \text{ or } (R'COO)_2O$$

in each of which R′ has the meaning defined in claim 1.

4. Process for the preparation of the derivatives of the general formula Ic :

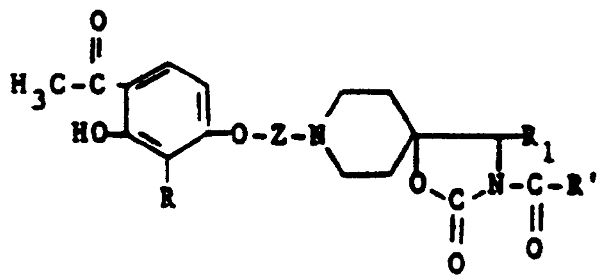

(Ic)

in which R, Z, R′ and $R_1$ have the meanings defined in claim 1, characterised in that a derivative of the general formula Ia according to claim 2 is reacted with half the stoichiometric amount of acylating agent R′COCl or $(R'COO)_2O$ as defined in claim 3.

5. Process for the preparation of the derivatives of the general formulae Id, Ie and If described below, characterised in that :

a) a derivative of the formula :

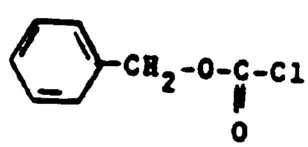

is reacted with derivative Ia defined in claim 2 to obtain the derivative of the general formula Id :

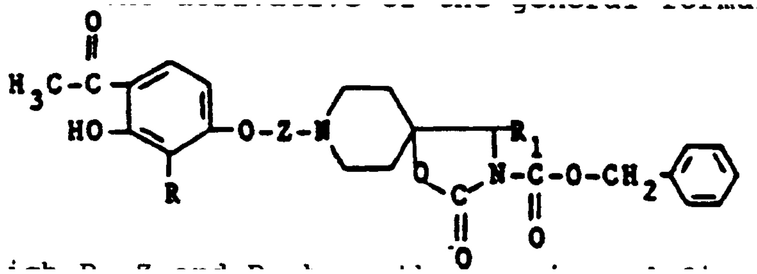

(Id)

in which R, Z and $R_1$ have the meanings defined in claim 1 ;

b) the derivative (Id) so obtained is acylated with an acylating agent R′COCl or $(R'COO)_2O$ according to claim 3, to obtain the derivative of the general formula Ie :

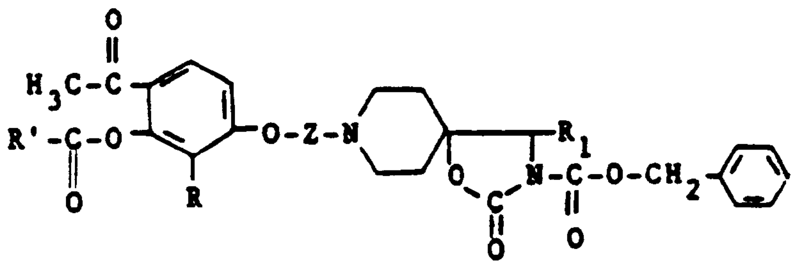

(Ie)

in which R, R′, Z and $R_1$ have the meanings defined in claim 1 ; and

c) the derivative Ie so obtained is subjected to hydrogenolysis using hydrogen, in the presence of palladium/carbon as catalyst, to obtain the derivative of the general formula If :

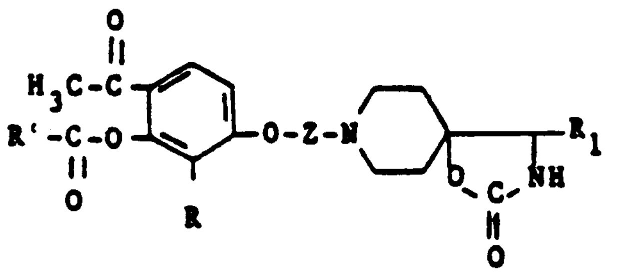

(If)

in which R, R′, Z and $R_1$ have the meanings defined in claim 1.

6. Process for the preparation of the derivatives of claim 1 corresponding to the general formula I in which X and Y together represent an ethenylene or ethylene chain substituted by a carboxy group, characterised in that derivative III according to claim 1 is condensed with a methyl- or ethyl-esterified form of the corresponding derivative II according to claim 1, and then the resulting derivative is hydrolysed using $NaHCO_3$ in order to free the carboxy function.